# EUROPEAN PATENT SPECIFICATION

(11) **EP 1 954 741 B1**
(45) Date of publication and mention of the grant of the patent: **22.12.2010**
(21) Application number: 06844561.8
(22) Date of filing: 27.11.2006
(51) Int. Cl.: C08G 64/16, C07C 233/00, C07C 321/00

(54) **DIHYDROXY AROMATIC COMPOUNDS, METHODS FOR PREPARATION POLYMERS MADE THEREFROM, AND METHOD OF POLYMER PREPARATION**
AROMATISCHE DIHYDROXY-VERBINDUNGEN, VERFAHREN ZUR HERSTELLUNG VON POLYMEREN DARAUS UND VERFAHREN ZUR POLYMERHERSTELLUNG
COMPOSES DIHYDROXY AROMATIQUES, PROCEDES DESTINES A LA PREPARATION DE POLYMERES A PARTIR DE CEUX-CI, ET PROCEDE DE PREPARATION D UN POLYMERE

(30) Priority: 29.11.2005 US 289210; 29.11.2005 US 289215
(43) Date of publication of application: 13.08.2008
(73) Proprietor: SABIC Innovative Plastics IP B.V., 4612 PX Bergen op Zoom (NL)
(72) Inventor: ARUMUGAM, Nagarajan, Erode District, Tamilnadu (IN); ARAKALI, Radhakrishna, Srinivasarao, Bangalore, Karnataka 560019 (IN); RAJ, Tilak, Karnataka 560 076 (IN); SAHOO, Binod, Orissa 755036 (IN); SCINDIA, Subash, Bangalore, Karnataka 560 033 (IN); LENS, Jan, Pleun, NL-4815 ED N-brabant (NL)
(74) Representative: Modiano, Micaela Nadia
(86) International application number: PCT/US2006/045456
(87) International publication number: WO 2007/064608

(56) References cited:
- WEN, YONG HONG; ZHANG, SHU SHENG; LIANG, JUAN; LI, XUE MEI: "N,N'-Bis(p-methoxyphenyl)-2,2'-(p-phenyle nedioxy)diacetamide" ACTA CRYSTALLOGRAPHICA, SECTION E: STRUCTURE REPORTS ONLINE, vol. E61, no. 1, 2005, pages O96-O97, XP002427919
- WEN, YONG HONG; ZHANG, SHU SHENG; LIANG, JUAN; LI, XUE MEI: "N,N'-Bis(p-methoxyphenyl)-2,2'-(m-phenyle nedioxy)diacetamide" ACTA CRYSTALLOGRAPHICA, SECTION E: STRUCTURE REPORTS ONLINE, vol. E60, no. 10, 2004, pages O1702-O1703, XP002427920
- WANG X; WEI T; CHEN J; LI J: "Phase transfer catalyzed synthesis of N,N'-diaryl-1,2-phenylene dioxydiacetamides" SYNTHETIC COMMUNICATIONS, vol. 26, no. 14, 1996, pages 2765-2773, XP009081779
- DATABASE CA [Online] CHEMICAL ABSTRACTS SERVICE, COLUMBUS, OHIO, US; HIKOSAKA, TAKAAKI ET AL: "Electrophotographic photoreceptor containing polycarbonate binder resin in photosensitive layer" XP002427928 retrieved from STN Database accession no. 126:67447 -& JP 08 248650 A (IDEMITSU KOSAN CO, JAPAN) 27 September 1996 (1996-09-27)

## Description

### BACKGROUND OF THE INVENTION

This disclosure relates to dihydroxy aromatic compounds, methods of preparing these compounds, polymers prepared using these compounds, and methods of preparing these polymers. More particularly this disclosure relates to polycarbonates prepared musing the dihydroxy aromatic compounds, methods of preparing the polycarbonate and polycarbonate resins, compositions comprising the polycarbonate and polycarbonate resins, and uses thereof.

Dihydroxy aromatic compounds are generally known to be useful in the preparation of polycarbonates that exhibit exceptional properties like high glass transition temperature (Tg), high refractive index (RI), chemical resistance, and barrier properties. Materials having higher Tg and higher RI properties are in great demand for use in various applications like automotives and optical media.

Accordingly, there is a continuing need for new compounds that will provide polymers with better chemical resistance and at the same time retain high Tg values.

### SUMMARY OF THE INVENTION

Disclosed herein are dihydroxy aromatic compounds having Formula (I), 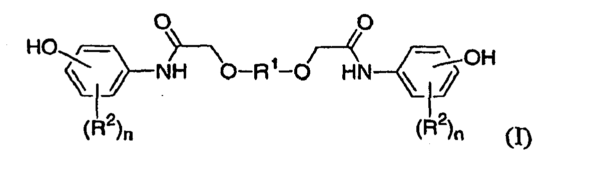 wherein R¹ is an aromatic functionality having 6 to 60 carbons, R² at each occurrence can be the same or different and is independently at each occurrence selected from the group consisting of a cyano functionality, a nitro functionality, a halogen, an aliphatic functionality having 1 to 10 carbons, a cycloaliphatic functionality having 3 to 10 carbons and an aromatic functionality having 6 to 10 carbons and further wherein "n" is an integer having a value of 0 to 4.

In another embodiment a process for producing the dihydroxy aromatic compounds of Formula (I) comprises reacting a compound of Formula (III) with a compound of Formula (IV) in the presence of a first base to produce a compound of Formula (V) 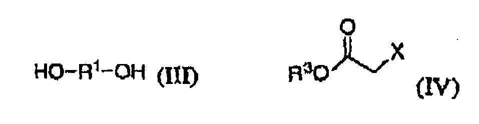 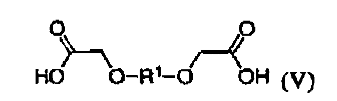 reacting the compound of Formula (V) in the presence of a first catalyst with a halogenating agent to provide the corresponding diacid halide having Formula (VI) 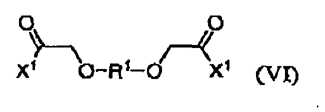 and reacting the compound of Formula (VI) with a compound of Formula (VII) in the presence of a second base to produce a compound of Formula (I) 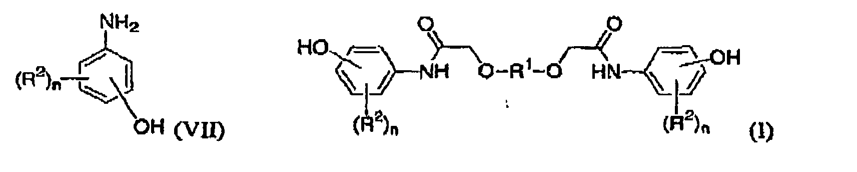 wherein R¹, R², and n are as described above, and X and X¹ are independently at each occurrence a halogen, R³ is selected from the group consisting of a hydrogen and an aliphatic functionality having 1 to 10 carbons.

In another embodiment a polymer comprises at least one structural unit derived from a dihydroxy aromatic compound of Formula (I).

In one embodiment a process for preparing a polymer comprising structural units derived from a dihydroxy aromatic compound of Formula (I) comprises polymerizing a dihydroxy aromatic compound of Formula (I).

Also disclosed herein are compositions comprising the polymer and articles comprising the polymer.

The above-described and other features are exemplified by the following detailed description.

### DETAILED DESCRIPTION OF THE INVENTION

Disclosed herein are dihydroxy aromatic compounds, and polymers prepared using these dihydroxy aromatic compounds. These polymers can be used in high heat and optical applications.

The singular forms "a", "an" and "the" include plural referents unless the context clearly dictates otherwise. All ranges disclosed herein are inclusive and combinable (for example ranges of "up to 25 weight (wt.) percent, with 5 wt. percent to 20 wt. percent desired," is inclusive of the endpoints and all intermediate values of the ranges of "5 wt. percent to 25 wt. percent").

"BPA" is herein defined as bisphenol A and is also known as 2,2-bis(4-hydroxyphenyl)propane, 4,4'-isopropylidenediphenol and p,p-BPA.

Unless otherwise specified, the term "cycloaliphatic functionality" designates cyclic aliphatic functionalities having a valence of at least one and comprising an array of atoms which is cyclic but which is not aromatic. A "cycloaliphatic functionality" may comprise one or more noncyclic components. For example, a cyclohexylmethyl group (C₆H₁₁CH₂) is a cycloaliphatic functionality, which comprises a cyclohexyl ring (the array of atoms which is cyclic but which is not aromatic) and a methylene group (the noncyclic component). The cycloaliphatic functionality may include heteroatoms such as nitrogen, sulfur, selenium, silicon and oxygen, or may be composed exclusively of carbon and hydrogen. For convenience, the term "cycloaliphatic functionality" is defined herein to encompass a wide range of functional groups such as alkyl groups, alkenyl groups, alkynyl groups, haloalkyl groups, conjugated dienyl groups, alcohol groups, ether groups, carboxylic acid groups, acyl groups (for example carboxylic acid derivatives such as esters and amides), amine groups and nitro groups. For example, the 4-methylcyclopent-1-yl group is a C₆ cycloaliphatic functionality comprising a methyl group, wherein the methyl group is a functional group which is an alkyl group. Similarly, the 2-nitrocyclobut-1-yl group is a C₄ cycloaliphatic functionality comprising a nitro group, wherein the nitro group is a functional group. A cycloaliphatic functionality may comprise one or more halogen atoms which may be the same or different. Exemplary cycloaliphatic functionalities comprise cyclopropyl, cyclobutyl, 1,1,4,4-tetramethylcyclobutyl, piperidinyl, 2,2,6,6-tetramethylpiperydinyl, cyclohexyl, and cyclopentyl.

As used herein, the term "aromatic functionality" refers to an array of atoms having a valence of at least one comprising at least one aromatic group. The array of atoms having a valence of at least one comprising at least one aromatic group may include heteroatoms such as nitrogen, sulfur, selenium, silicon and oxygen, or may be composed exclusively of carbon and hydrogen. As used herein, the term "aromatic functionality" includes but is not limited to, phenyl, pyridyl, furanyl, thienyl, naphthyl, phenylene, and biphenyl functionalities. The aromatic functionality may also include nonaromatic components. For example, a benzyl group is an aromatic functionality that comprises a phenyl ring (the aromatic group) and a methylene group (the nonaromatic component). Similarly a tetrahydronaphthyl functionality is an aromatic functionality comprising an aromatic group (C₆H₃) fused to a nonaromatic component (CH₂)₄. For convenience, the term "aromatic functionality" is defined herein to encompass a wide range of functional groups such as alkyl groups, haloalkyl groups, haloaromatic groups, alcohol groups, ether groups, carboxylic acid groups, acyl groups (for example carboxylic acid derivatives such as esters and amides), amine groups and nitro groups. For example, the 4-methylphenyl functionality is a C₇ aromatic functionality comprising a methyl group, wherein the methyl group is a functional group which is an alkyl group. Similarly, the 2-nitrophenyl group is a C₆ aromatic functionality comprising a nitro group, wherein the nitro group is a functional group. Aromatic functionalities include halogenated aromatic functionalities. Exemplary aromatic functionalities include, but are not limited to, phenyl, 4-trifluoromethylphenyl, 4-chloromethylphen-1-yl, 3-trichloromethylphen-1-yl (3-CCl₃Ph-), 4-(3-bromoprop-1-yl)phen-1-yl (4-BrCH₂CH₂CH₂Ph-),4-aminophen-1-yl (4-H₂NPh-), 4-hydroxymethylphen-l-yl (4-HOCH₂Ph-), 4-methylthiophen-1-yl (4-CH₃SPh-), 3-methoxyphen-1-yl and 2-nitromethylphen-1-yl (2-NO₂CH₂Ph) and naphthyl.

As used herein the term "aliphatic functionality" refers to a linear or branched array of atoms that is not cyclic and has a valence of at least one. Aliphatic functionalities are defined to comprise at least one carbon atom. The array of atoms may include heteroatoms such as nitrogen, sulfur, silicon, selenium and oxygen or may be composed exclusively of carbon and hydrogen. For convenience, the term "aliphatic functionality" is defined herein to encompass, as part of the "linear or branched array of atoms which is not cyclic" a wide range of functional groups such as alkyl groups, haloalkyl groups, alcohol groups, ether groups, carboxylic acid groups, acyl groups (for example carboxylic acid derivatives such as esters and amides), amine groups and nitro groups. For example, the 4-methylpent-1-yl is a C₆ aliphatic functionality comprising a methyl group, wherein the methyl group is a functional group which is an alkyl group. Similarly, the 4-nitrobut-1-yl group is a C₄ aliphatic functionality comprising a nitro group, wherein the nitro group is a functional group. An aliphatic functionality may be a haloalkyl group which comprises one or more halogen atoms which may be the same or different. Exemplary aliphatic functionalities include, but are not limited to, methyl, ethyl, propyl, isopropyl, butyl, isobutyl, pentyl, isopentyl, trifluoromethyl, bromodifluoromethyl, chlorodifluoromethyl, chloromethyl, trichloromethyl, bromoethyl, 2- hexyl, hexamethylene, hydroxymethyl (i.e., CH₂OH), mercaptomethyl (CH₂SH), methylthio (SCH₃), methylthiomethyl (CH₂SCH₃), methoxy, methoxycarbonyl (CH₃OCO), nitromethyl (CH₂NO₂) and thiocarbonyl.

In one embodiment the dihydroxy aromatic compound comprises compounds of Formula (X), Formula (XI), Formula (XII) or Formula (XIII), 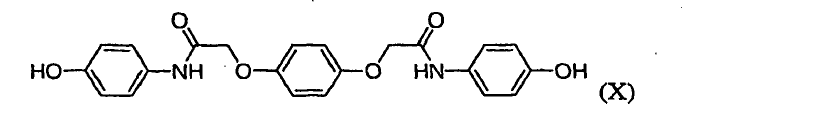 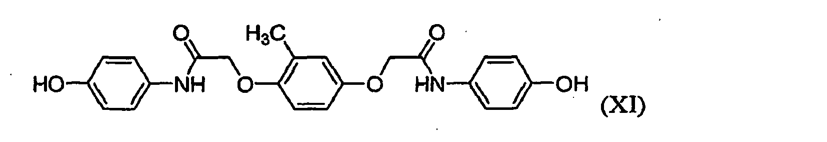 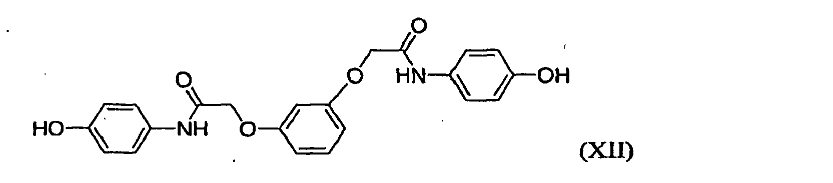 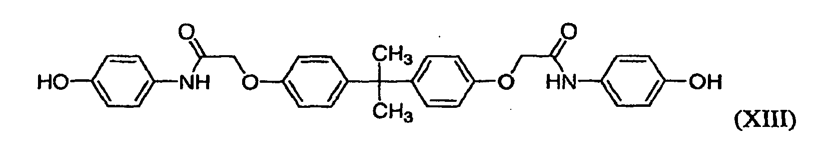 wherein R¹ is derived from hydroquinone in Formula (X), from methyl hydroquinone in Formula (XI), from resorcinol in Formula (XII) and from bisphenol A in Formula (XIII), and "n" in Formula (X), Formula (XI), Formula (XII) and Formula (XIII) is 0. The compounds of Formula (X), Formula (XI), Formula (XII) and Formula (XIII) may hereinafter also be referred to as N-(4-hydroxy-phenyl)-2-[4-[(4-hydroxyphenylcarbomoyl)-methoxy]-phenoxy]-acetamide; N-(4-hydroxy-phenyl)-2-[4-[(4-hydroxy-phenylcarbomoyl)-methoxy]-2-methyl-phenoxy]-acetamide, N-(4-hydroxyphenyl)-2-(3-[(4-hydroxyphenylcarbomoyl)-methoxy]-phenoxy)-acetamide and N-(4-hydroxy-phenyl)-2-(4-(1-methyl-1-[4-(4-hydroxyphenlycarbomyl-methoxy)-phenyl]-ethyl)-phenoxy)-acetamide respectively.

The process for making the dihydroxy aromatic compound of Formula (I) comprises the following steps. The first step comprises reacting a compound of Formula (III) with a compound of Formula (IV) in the presence of a first base to produce a compound of Formula (V) 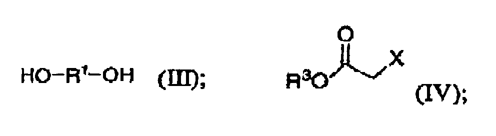 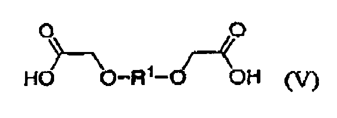 wherein R¹, R³ and X have the same meaning as defined above.

Suitable compounds of Formula (III) can be represented by bisphenol compounds having Formula (XIV), 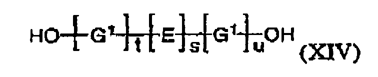 wherein each G¹ is independently at each occurrence an aromatic functionality having 6 to 20 carbons; E is independently at each occurrence, a cycloaliphatic functionality having 3 to 20 carbons, an aromatic functionality having 6 to 20 carbons, an aliphatic functionality having 1 to 20 carbons, a sulfur-containing linkage (for example, -S-, - SO-, -SO₂-), a selenium-containing linkage (for example, -Se-, -SeO-, -SeO₂-), a phosphorus-containing linkage, or an oxygen atoms: "t" is a number greater than or equal to one and less than or equal to 10,000; "s" is either zero or one; "u" is a number including zero to 10,000. When "s" equals zero then [G¹]ₜ is bonded to [G¹]ᵤ.

Exemplary compounds having Formula (XIV) include, but are not limited to, 1,1-bis(4-hydroxyphenyl)cyclopentane; 2,2-bis(3-allyl-4-hydroxyphenyl)propane, 2,2-bis(2-t-butyl-4-hydroxy-5-methylphenyl)propane; 2,2-bis(3-t-butyl-4-hydroxy-6-methylphenyl)propane; 2,2-bis(3-t-butyl-4-hydroxy-6-methylphenyl)butane; 1,3 - bis[4-hydroxyphenyl-1-(1-methylethylidine)]benzene-, 1,4-bis[4-hydroxyphenyl-1-(1-methylethylidine)]benzene; 1,3-bis[3-t-butyl-4-hydroxy-6-methylphenyl-1-(1-methylethylidine)]benzene; 1,4-bis[3-t-butyl-4-hydroxy-6-methylphenyl-1-(1-methylethylidine)]benzene; 4,4'-biphenol; 2,2-bis(3-methyl-4-hydroxyphenyl)propane; 1,1-bis(4-hydroxyphenyl)norbornane; 1,2-bis(4-hydroxyphenyl)ethane; bis(4-hydroxyphenyl) sulfide; 4,4'-oxydiphenol; 2-phenyl-3,3-bis(4-hydroxyphenyl)phthalimidine; 2,4'-dihydroxydiphenylmethane; 2-bis(2-hydroxyphenyl)methane; bis(4-hydroxyphenyl)methane; bis(4-hydroxy-5-nitrophenyl)methane; bis(4-hydroxy-2,6-dimethyl-3-methoxyphenyl)methane; 1,1-bis(4-hydroxyphenyl)ethane; 2,2-bis(4-hydroxyphenyl)propane (bisphenol A); 1,1-bis(4-hydroxyphenyl)propane; 2,2-bis(4-hydroxy-3-methylphenyl)propane; 2,2-bis(4-hydroxy-3-isopropylphenyl)propane; 2,2-bis(3-t-butyl-4-hydroxyphenyl)propane; 2,2-bis(3-phenyl-4-hydroxyphenyl)propane; 2,2-bis(3,5-dimethyl-4-hydroxyphenyl)propane; 2,2-bis(3,5-disopropyl-4-hydroxyphenyl)propane; 2,2-bis(3,5-di-t-butyl-4-hydroxyphenyl)propane; 2,2-bis(3,5-diphenyl-4-hydroxyphenyl)propane; 2,2-bis(4-hydroxy-2,3,5,6-tetramethylphenyl)propane; 2,2-bis(4-hydroxy-3-ethylphenyl)propane; 2,2-bis(4-hydroxy-3,5-dimethylphenyl)propane; 1,1-bis(4-hydroxyphenyl)cyclohexylmethane; 2,2-bis(4-hydroxyphenyl)-1-phenylpropane; 1,1-bis(4-hydroxyphenyl)cyclohexane; 1,1-bis(4-hydroxy-3-methylphenyl)cyclohexane; 1,1-bis (4-hydroxy-3-isopropylphenyl)cyclohexane; 1,1-bis(3-t-butyl-4-hydroxyphenyl)cyclohexane; 1,1-bis(3-phenyl-4-hydroxyphenyl)cyclohexane; 1,1-bis(3,5-dimethyl-4-hydroxyphenyl)cyclohexane; 4,4'-[1-methyl-4-(1-methyl-ethyl)-1,3-cyclohexandiyl]bisphenol (1,3 BHPM); 4-[1-[3-(4-hydroxyphenyl)-4-methylcyclohexyl]-1-methyl-ethyl]-phenol (2,8-BHPM); 1,1-bis(3,5-disopropyl-4-hydroxyphenyl)cyclohexane; 1,1-bis(3,5-di-t-butyl-4-hydroxyphenyl)cyclohexane; 1,1-bis(3,5-diphenyl-4-hydroxyphenyl)cyclohexane; 1,1-bis(4-hydroxy-2,3,5,6-tetramethylphenyl)cyclohexane; 1,1-bis(4-hydroxyphenyl)-3,3,5-trimethylcyclohexane;; 1,1-bis(4-hydroxy-3-methylphenyl)-3,3,5-trimethylcyclohexane; 1,1-bis(4-hydroxy-3-isopropylphenyl)-3,3,5-trimethylcyclohexane; 1,1-bis(3-t-butyl-4-hydroxyphenyl)-3,3,5-trimethylcyclohexane; 1,1-bis(3-phenyl-4-hydroxyphenyl)-3,3,5-trimethylcyclohexane; 1,1-bis(3,5-dimethyl-4-hydroxyphenyl)-3,3,5-trimethylcyclohexane; 1,1-bis(3,5-disopropyl-4-hydroxyphenyl)-3,3,5-trimethylcyclohexane; 1,1-bis(3,5-di-t-butyl-4-hydroxyphenyl)-3,3,5-trimethylcyclohexane; 1,1-bis(3,5-diphenyl-4-hydroxyphenyl)-3,3,5-trimethylcyclohexane; 1,1 -bis(4-hydroxy-2,3,5,6-tetramethylphenyl)-3,3 ,5-trimethylcyclohexane; 4,4-bis(4-hydroxyphenyl)heptane; 1,1-bis(4-hydroxyphenyl)decane; 1,1-bis(4-hydroxyphenyl)cyclododecane; 1,1-bis(3,5-dimethyl-4-hydroxyphenyl)cyclododecane; 4,4'dihydroxy-1,1-biphenyl; . 4,4'-dihydroxy-3,3' -dimethyl-1,1 -biphenyl; 4,4'-dihydroxy-3,3'-dioctyl-1,1-biphenyl; 4,4'-(3,3,5-trimethylcyclohexylidene)diphenol; 4,4'-bis(3,5-dimethyl)diphenol; 4,4'-dihydroxydiphenylether; 4,4'-dihydroxydiphenylthioether; 1,3-bis(2-(4-hydroxyphenyl)-2-propyl)benzene; 1,3-bis(2-(4-hydroxy-3-methylphenyl)-2-propyl)benzene; 1,4-bis(2-(4-hydroxyphenyl)-2-propyl)benzene; 1,4-bis(2-(4-hydroxy-3-methylphenyl)-2-propyl)benzene; 2,4'-dihydroxyphenyl sulfone; 4,4'-dihydroxydiphenylsulfone (BPS); bis(4-hydroxyphenyl)methane; 2,6-dihydroxy naphthalene; hydroquinone; methyl hydroquinone; resorcinol; C₁₋₃ alkyl-substituted resorcinols; 3-(4-hydroxyphenyl)-1,1,3-trimethylindan-5-ol; 1-(4-hydroxyphenyl)-1,3,3-trimethylindan-5-ol; 4,4-dihydroxydiphenyl ether; 4,4-dihydroxy-2,5-dihydroxydiphenyl ether; 4,4-thiodiphenol; 2,2,2',2'-tetrahydro-3,3,3',3'-tetramethyl-1,1'-spirobi[1H-indene]-6,6'-diol; and combinations of two or more of the foregoing.

The amount of the compound of Formula (IV) employed in the reaction can be 1 mole to 6 moles per mole of compound of Formula (III) employed. Within this range, the amount may be greater than or equal to 1.5 moles, or, more specifically, greater than or equal to 2 moles. Also within this range the amount may be less than or equal to 4 moles, or, more specifically, less than or equal to 2.5 moles.

One exemplary first base comprises alkali metal hydroxide or alkaline earth metal hydroxide. Another exemplary first base comprises alkali metal carbonate or alkaline earth metal carbonate in combination with alkali metal halide or alkaline earth metal halide.

Specific examples of suitable alkali metal hydroxides or alkaline earth metal hydroxides that can be employed as the first base include, but are not limited to, sodium hydroxide, potassium hydroxide, lithium hydroxide, rubidium hydroxide, cesium hydroxide, calcium hydroxide, magnesium hydroxide and combinations of two or more of the foregoing hydroxides. In one embodiment the first base comprises sodium hydroxide. The alkali metal hydroxides or alkaline earth metal hydroxides can be added as an aqueous solution or as a solid. The amount of first base employed when the first base is an alkali metal hydroxide or an alkaline earth metal hydroxide can be 1 mole to 10 moles per mole of the compound of Formula (III) employed. Within this range the amount may be greater than or equal to 1.5 moles, or, more specifically, greater than or equal to 2 moles. Also within this range the amount may be less than or equal to 5 moles, or, more specifically, less than or equal to 3 moles.

Suitable alkali metal carbonates or alkaline earth metal carbonates that can be used in combination with alkali metal halides or alkaline earth metal halides include, but are not limited to, potassium carbonate, sodium carbonate, calcium carbonate and magnesium carbonate, and combinations of two or more of the foregoing carbonates. The amount of alkali metal carbonate or alkaline earth metal carbonate used in the reaction can be 1 mole to 5 moles per mole of the compound of Formula (III) employed. Within this range the amount may be greater than or equal to 2 moles, or, more specifically, greater than or equal to 2.5 moles. Also within this range the amount may be less than or equal to 4 moles, or, more specifically, less than or equal to 3 moles.

Suitable alkali metal halides or alkaline earth metal halides that can be used in combination with alkali metal carbonate or alkaline earth metal carbonate include, but are not limited to, sodium iodide, potassium iodide and a combination of sodium iodide and potassium iodide. The amount of alkali metal halide or alkali earth metal halide employed in the reaction can be 1 mole to 5 moles per mole of the compound of Formula (III) employed. Within this range the amount may be greater than or equal to 2 moles, or, more specifically, greater than or equal to 2.5 moles. Also within this range the amount may be less than or equal to 4 moles, or, more specifically, less than or equal to 3 moles.

In one embodiment the alkali metal carbonate is potassium carbonate and the alkali metal halide is sodium iodide. The amount of alkali metal carbonate or alkaline earth metal carbonate can be 1 mole to 3 moles per mole of alkali metal halide or alkaline earth metal halide. Within this range the amount may be greater than or equal to 1.5 moles, or, more specifically, greater than or equal to 2 moles. Also within this range the amount may be less than or equal to 2.75 moles, or, more specifically, less than or equal to 2.5 moles.

Specific examples of solvents that can be employed in the reaction of the compound of Formula (III) with the compound of Formula (IV) include, but are not limited to, water, acetone, dimethylformamide (DMF), tetrahydrofuran (THF), diphenylether, dimethylsulfoxide (DMSO) and combinations of two or more of the foregoing solvents. In one embodiment the solvent employed comprises water, acetone or a combination of water and acetone. In certain embodiments the amount of solvent employed in the reaction of the compound of Formula (III) with the compound of Formula (IV) can be 1 liter to 10 liters per mole of compound of Formula (III). Within this range the amount may be greater than or equal to 3 liters, or, more specifically, greater than or equal to 5 liters. Also within this range the amount may be less than or equal to 8 liters, or, more specifically, less than or equal to 6 liters.

The temperature at which the reaction of the compound of Formula (III) with the compound of Formula (IV) proceeds can be 30°C to 100°C. Within this range the temperature may be greater than or equal to 40°C, or, more specifically, greater than or equal to 60°C. Also within this range the temperature may be less than or equal to 90°C, or, more specifically, less than or equal to 80°C. The time taken for the reaction of the compound of Formula (III) with the compound of Formula (IV) can be 5 hours to 50 hours. Within this range the time may be greater than or equal to 10 hours, or, more specifically, greater than or equal to 20 hours. Also within this range the time may be less than or equal to 45 hours, or, more specifically, less than or equal to 40 hours.

The reaction of the compound of Formula (III) with the compound of Formula (IV) directly provides the compound of Formula (V) when R³ is H. When R³ is an aliphatic functionality the reaction of a compound of Formula (III) with the compound of Formula (IV) under the conditions described above provides a diester having the formula 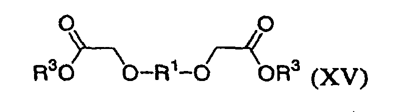 wherein R¹ is as described above.

The diester of Formula (XV) can be hydrolyzed in the presence of an alkali metal hydroxide or alkali metal carbonate to provide the corresponding diacid compound of Formula (V). Exemplary alkali metal hydroxide or alkali metal carbonates include but are not limited to, sodium hydroxide, potassium hydroxide, lithium hydroxide, cesium hydroxide, sodium carbonate, potassium carbonate and combinations of two or more of the foregoing hydroxides.

The amount of alkali metal hydroxide or alkali metal carbonate employed in the hydrolysis can be 1 mole to 8 moles per mole of diester of Formula (XV). Within this range the amount may be greater than or equal to 2 moles, or, more specifically, greater than or equal to 3 moles. Also within this range the amount may be less than or equal to 6 moles, or, more specifically, less than or equal to 5 moles.

Suitable solvent that can be used in the hydrolysis reaction of the diester of Formula (XV) to the corresponding diacid of Formula (V) includes but is not limited to, ethanol, methanol, tetrahydrofuran and dioxane. The amount of solvent employed in the hydrolysis reaction can be 0.5 liters to 4 liters per mole of diester of Formula (XV). Within this range the amount may be greater than or equal to 1 liter, or, more specifically, greater than or equal to 1.5 liters. Also within this range the amount may be less than or equal to 3 liters, or, more specifically, less than or equal to 2.5 liters.

The temperature of the hydrolysis reaction of the compound of Formula (XV) can be 25°C to 80°C. Within this range the temperature may be greater than or equal to 30°C, or, more specifically, greater than or equal to 40°C. Also within this range the temperature may be less than or equal to 70°C, or, more specifically, less than or equal to 60°C. The time taken for the hydrolysis reaction can be 10 hours to 24 hours. Within this range the time may be greater than or equal to 12 hours, or, more specifically, greater than or equal to 16 hours. Also within this range the time may be less than or equal to 20 hours, or, more specifically, less than or equal to 18 hours.

The compound of Formula (V) is reacted in the presence of a first catalyst with a halogenating agent to provide the corresponding diacid halide having Formula (VI) 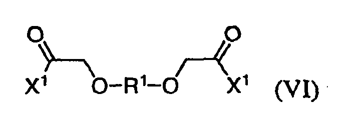 wherein R¹ and X¹ are as described above.

Exemplary halogenating agents include, but are not limited to, thionyl chloride, phosphorous trichloride, phosphorous pentachloride, phosphorous pentabromide, thionyl bromide, phosphorous tribromide, and oxalyl chloride. In one embodiment the halogenating agent is thionyl chloride.

The amount of halogenating agent employed in the reaction can be 2 moles to 10 moles per mole of compound of Formula (V). Within this range the amount may be greater than or equal to 2.5 moles, or, more specifically, greater than or equal to 3 moles. Also within this range the amount may be less than or equal to 8 moles, or, more specifically, less than or equal to 4 moles.

Suitable first catalysts that can be employed in the halogenation of the compound of Formula (V) or include, but are not limited to, dimethylformamide, dimethylacetamide, dimethylaminopyridine, dimethylaniline, diethylamine and combinations of two or more of the foregoing catalysts.

The amount of first catalyst employed in the reaction can be 0.01 moles to 0.1 moles per mole of compound of Formula (V). Within this range the amount may be greater than or equal to 0.02 moles, or, more specifically, greater than or equal to 0.03 moles. Also within this range the amount may be less than or equal to 0.08 moles, or, more specifically, less than or equal to 0.05 moles.

Suitable solvents that can be employed in the halogenation of the compound of Formula (V) include, but are not limited to, toluene, xylene, chloroform, methylene dichloride, ethylene dichloride and carbon tetrachloride. In one embodiment the solvent used is ethylene dichloride.

In certain embodiments the amount of solvent employed in the halogenation of the compound of Formula (V) can be 1.5 liters to 3.0 liters per mole of compound of Formula (V). Within this range the amount may be greater than or equal to 1.75 liters, or, more specifically, greater than or equal to 2.0 liters. Also within this range the amount may be less than or equal to 2.75 liters, or, more specifically, less than or equal to 2.25 liters.

The temperature of the halogenation reaction of the compound of Formula (V) can be 40°C to 140°C. Within this range the temperature may be greater than or equal to 60°C, or, more specifically, greater than or equal to 80°C. Also within this range the temperature may be less than or equal to 120°C, or, more specifically, less than or equal to 90°C. The time taken for the halogenation reaction of the compound of Formula (V) can be 1 hour to 10 hours. Within this range the time may be greater than or equal to 3 hours, or, more specifically, greater than or equal to 4 hours. Also within this range the time may be less than or equal to 8 hours, or, more specifically, less than or equal to 6 hours.

The compound of Formula (VI) is reacted with a compound of Formula (VII) in the presence of a second base to produce a dihydroxy aromatic compound of Formula (I) 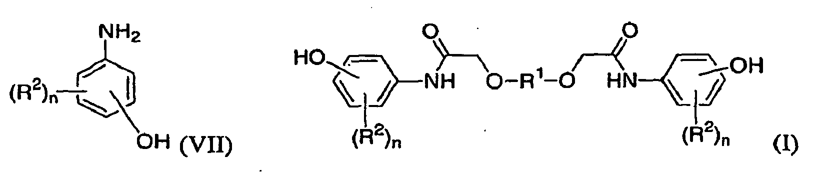 wherein R¹, R² and "n" have the same meaning as defined above.

Suitable compounds having Formula (VII) include, but are not limited to, 4-aminophenol, 3-aminophenol, 2-methyl-4-aminophenol, 4-chloro-2-aminophenol, 2-amino-4-chlorophenol, 2-amino-5-chlorophenol and 4-amino-2-nitrophenol. In one embodiment the compound of Formula (VII) comprises 4-aminophenol having Formula (IX) shown below.

The amount of the compound of Formula (VII) employed in the reaction can be 1 mole to 5 moles per mole of compound having Formula (VI). Within this range the amount may be greater than or equal to 2 moles, or, more specifically, greater than or equal to 2.5 moles. Also within this range the amount may be less than or equal to 4 moles, or, more specifically, less than or equal to 3 moles.

Suitable second bases include, but are not limited to, organic or inorganic bases having sufficient strength to remove a proton from the amine group of the compound of Formula (VII) without removing substantial amounts of protons from the hydroxy group. Specific examples of inorganic bases include alkali metal hydroxides or alkaline earth metal hydroxides including, but not limited to, sodium hydroxide, potassium hydroxide, lithium hydroxide, rubidium hydroxide cesium hydroxide, calcium hydroxide, magnesium hydroxide and combinations of two or more of the foregoing alkali metal hydroxides. In one embodiment the second base comprises sodium hydroxide. Specific examples of organic bases include but are not limited to, triethylamine, piperidine, piperidine, ethyldiisopropylamine, triethylamine, pyridine, pyrrolidone, morpholine, sodium carbonate, potassium carbonate, sodium methylate, potassium methylate and combinations of two or more of the foregoing. The second base can be added as an aqueous solution or as a solid.

The amount of second base employed in the reaction can be 0.5 moles to 3 moles per mole of compound of Formula (VII) employed. Within this range the amount may be greater than or equal to 1 mole, or, more specifically, greater than or equal to 1.5 moles. Also within this range the amount may be less than or equal to 2.5 moles, or, more specifically, less than or equal to 2 moles.

Specific examples of suitable solvents that can be employed in the reaction of the compound of Formula (VI) in the presence of a second base with a compound of Formula (VII) include, but are not limited to, dimethylformamide, dimethylsulfoxide, dimethylacetamide, tetrahydrofuran, dioxane, ethylene dichloride, methylene dichloride, chloroform, carbon tetrachloride and combinations of two or more of the foregoing. In one embodiment the solvent employed comprises dimethylformamide, dimethylacetamide, or a combination of the two. The amount of solvent employed in the reaction of compound having the compound of Formula (VI) in the presence of a second base with a compound of Formula (VII) can be 0.5 liters to 3 liters per mole of having the compound of Formula (VII). Within this range the amount may be greater than or equal to 1 liter, or, more specifically, greater than or equal to 1.5 liters. Also within this range the amount may be less than or equal to 2.5 liters, or, more specifically, less than or equal to 2 liters.

The temperature of the reaction of a compound of Formula (VI) in the presence of a second base with a compound of Formula (VII) can be 0°C to 40°C. Within this range the temperature may be greater than or equal to 5°C, or, more specifically, greater than or equal to 10°C. Also within this range the temperature may be less than or equal to 30°C, or, more specifically, less than or equal to 25°C. The time for the reaction of compound of Formula (VI) in the presence of a second base with a compound of Formula (VII) can be 5 hours to 40 hours. Within this range the time may be greater than or equal to 10 or, more specifically, greater than or equal to 20 hours. Also within this range the time may be less than or equal to 35 hours, or, more specifically, less than or equal to 30 hours.

In one embodiment a process for producing the dihydroxy aromatic compounds of Formula (II) comprises reacting a compound of Formula (III) with a compound of Formula (IV) in the presence of a first base to produce a compound of Formula (V) 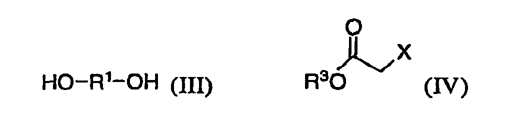 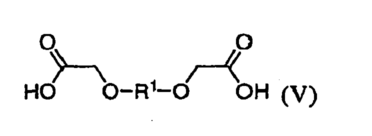 reacting the compound of Formula (V) in the presence of a first catalyst with a chlorinating agent to provide the corresponding diacid chloride having Formula (VIII) 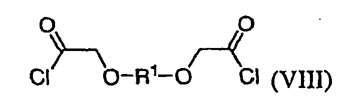 and reacting the compound of Formula (VIII) in the presence of a second base with 4-aminophenol (Formula (IX)) to produce a compound of Formula (II) 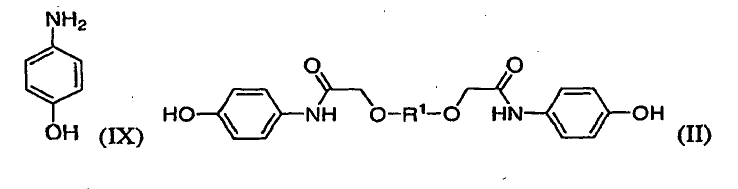 wherein R¹ is derived from hydroquinone, methyl hydroquinone, resorcinol or bisphenol A, R³ is hydrogen or methyl and "n" is an integer having a value 0 to 4.

In one embodiment a composition comprises a dihydroxy aromatic compound of Formula (I) 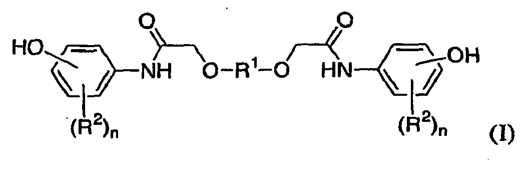 wherein R¹, R², and "n" are defined as above.

In one embodiment a polymer comprises at least one structural unit derived from a dihydroxy aromatic compound of Formula (I) 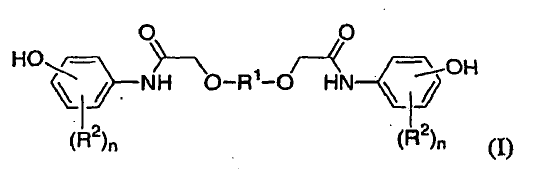 wherein R¹, R², and "n" are defined as above. A variety of polymers may comprise the structural units derived from the dihydroxy aromatic compound of Formula (I), including, but not limited to, polycarbonate, polyester, copolyester-polycarbonate, polyurethane, and epoxide containing polymers.

In one embodiment a polycarbonate comprises at least one structural unit derived from a dihydroxy aromatic compound of Formula (II) 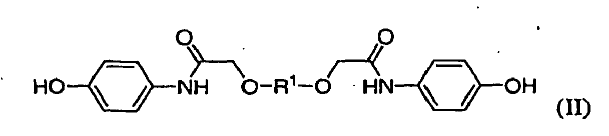 wherein R¹ is an aromatic divalent functionality derived from hydroquinone, resorcinol, methyl hydroquinone or bisphenol A.

"Polycarbonates" and "polycarbonate resins" as used herein are polymers comprising structural units represented by Formula (XIX): 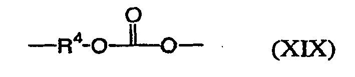 wherein at least 60 percent of the total number of R⁴ groups are aromatic functionalities and the balance thereof are aliphatic, alicyclic, or aromatic functionalities and further wherein at least one R⁴ group is derived from a dihydroxy aromatic compound of Formula (I).

The aromatic functionality may comprise a functionality of the Formula (XX):

-A¹-Y¹-A²- (XX)

wherein each of A¹ and A² is a monocyclic divalent aromatic functionality and Y¹ is a bridging functionality having one or two atoms that separate A¹ from A². In an exemplary embodiment, one atom separates A¹ from A². Illustrative non-limiting examples of functionalities of this type are -O-, -S-, -S(O)-, -S(O₂)-, -C(O)-, methylene, cyclohexyl-methylene, 2-[2.2.1]-bicycloheptylidene, ethylidene, isopropylidene, neopentylidene, cyclohexylidene, cyclopentadecylidene, cyclododecylidene, and adamantylidene. In one embodiment, the bridging functionality Y¹ may be a hydrocarbon group or a saturated hydrocarbon group such as methylene, cyclohexylidene, or isopropylidene.

"Polyesters" as used herein may comprise repeating structural units of the Formula (XXI) 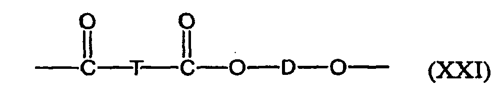 wherein D is derived from a divalent functionality derived from a dihydroxy compound, and may be, for example, a cycloaliphatic functionality having 6 to 10 carbon atoms, an aromatic functionality having 6 to 20 carbon atoms or an aliphatic functionality having 2 to 10 carbon atoms and wherein at least one of D are derived from a dihydroxy aromatic compound of Formula (I); and T is a divalent functionality derived from a dicarboxylic acid, and may be, for example, a cycloaliphatic functionality having 6 to 10 carbon atoms, an aromatic functionality having 6 to 20 carbon atoms or an aliphatic functionality having 2 to 10 carbon atoms.

In one embodiment, D comprises an aliphatic functionality having 2 to 10 carbon atoms. In another embodiment, D may be derived from an aromatic dihydroxy compound of Formula (XXII): 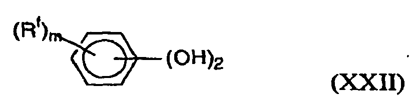 wherein each R^{f} is independently a halogen atom, an aliphatic functionality having 1 to 10 carbon atoms and "m" is an integer having a value 0 to 4. Examples of compounds that may be represented by the Formula (XXII) include, but are not limited to resorcinol, substituted resorcinol compounds such as 5-methyl resorcinol, 5-ethyl resorcinol, 5-propyl resorcinol, 5-butyl resorcinol, 5-t-butyl resorcinol, 5-phenyl resorcinol, 5-cumyl resorcinol, 2,4,5,6-tetrafluoro resorcinol, 2,4,5,6-tetrabromo resorcinol, or the like; catechol; hydroquinone; substituted hydroquinones such as 2-methyl hydroquinone, 2-ethyl hydroquinone, 2-propyl hydroquinone, 2-butyl hydroquinone, 2-t-butyl hydroquinone, 2-phenyl hydroquinone, 2-cumyl hydroquinone, 2,3,5,6-tetramethyl hydroquinone, 2,3,5,6-tetra-t-butyl hydroquinone, 2,3,5,6-tetrafluoro hydroquinone, 2,3,5,6-tetrabromo hydroquinone, or the like; or combinations comprising at least one of the foregoing compounds.

In one embodiment "T" is a divalent functionality derived from a dicarboxylic acid compound of Formula (XXIII) 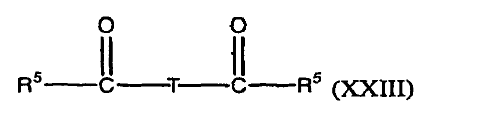 wherein R⁵ is independently at each occurrence hydroxy, chloro, or OR⁶, wherein R⁶ is independently at each occurrence selected from the group consisting of an aliphatic functionality having 1 to 10 carbons, a cycloaliphatic functionality having 3 to 10 carbons, and an aromatic functionality having 6 to 20 carbons. In one embodiment the divalent functionality "T" comprises a cycloaliphatic functionality having 6 to 10 carbon atoms, an aromatic functionality having 6 to 10 carbon atoms, or an aliphatic functionality having 2 to 10 carbon atoms.

Examples of dicarboxylic acids that may be used to prepare the polyesters include, but are not limited to 1,6-hexanedioic acid, phthalic acid, isophthalic acid, terephthalic acid, fumaric acid, maleic acid, azelaic acid, glutaric acid, adipic acid, suberic acid, sebacic acid, malonic acid, succinic acid, 1,2-di(p-carboxyphenyl)ethane, 4,4'-dicarboxydiphenyl ether, 4,4'-bisbenzoic acid, and combinations comprising two or more of the foregoing acids. Acids containing fused rings can also be present, such as in 1,4-, 1,5-, or 2,6-naphthalenedicarboxylic acids. Specific dicarboxylic acids are terephthalic acid, isophthalic acid, naphthalene dicarboxylic acid, cyclohexane dicarboxylic acid, or mixtures thereof. A specific dicarboxylic acid comprises a mixture of isophthalic acid and terephthalic acid wherein the weight ratio of terephthalic acid to isophthalic acid is 10:1 to 0.2:9.8. In another specific embodiment, D comprises a C₂₋₆ alkylene functionality and T comprises p-phenylene, m-phenylene, naphthalene, a divalent cycloaliphatic functionality, or acombination of two or more of the foregoing. This class of polyester includes the poly(alkylene terephthalates).

"Copolyester-polycarbonate" or "copolyestercarbonate" or "polyester carbonate" as used herein are copolymers containing recurring carbonate units of Formula (XIX) in addition to the repeating units of Formula (XXI) as defined above. In one embodiment either repeating carbonate units of Formula (XIX) or repeating units of Formula (XXI) or repeating units of both Formula (XIX) and Formula (XXI) comprise at least one structural unit derived from the dihydroxy aromatic compound of Formula (I).

Polyurethane as used herein are polymers containing recurring units having Formula (XXIV) 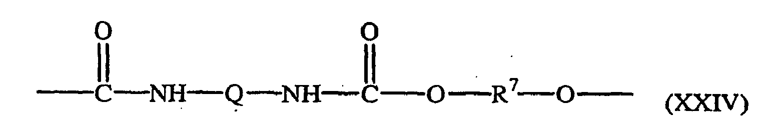 wherein O-R⁷-O is a divalent functionality derived from a dihydroxy compound or a polyhydroxy compound; wherein at least one of R⁷ is derived from a dihydroxy aromatic compound of Formula (I); and wherein "Q" is a divalent functionality derived from a diisocyanate compound, having Formula (XXV)

Q(NCO)₂ (XXV)

wherein Q comprises a divalent aliphatic functionality having 2 to 28 carbons, a divalent cycloaliphatic functionality having 4 to 15 carbons, or a divalent aromatic functionality having 6 to 15 carbons.

Examples of dicarboxylic acids that may be used to prepare the polyurethanes include, but are not limited to, 1,6-hexanedioc acid, phthalic acid, isophthalic acid, terephthalic acid, fumaric acid, maleic acid, azelaic acid, glutaric acid, adipic acid, suberic acid, sebacic acid, malonic acid, succinic acid, 1,2-di(p-carboxyphenyl)ethane, 4,4'-dicarboxydiphenyl ether, 4,4'-bisbenzoic acid, and mixtures comprising at least one of the foregoing acids. Acids containing fused rings can also be present, such as in 1,4-, 1,5-, or 2,6-naphthalenedicarboxylic acids. In one embodiment the dicarboxylic acids used are 1,6-hexanedioc acid terephthalic acid, isophthalic acid, naphthalene dicarboxylic acid, cyclohexane dicarboxylic acid, or combinations of two or more of the foregoing dicarboxylic acids.

Suitable examples of diisocyanate include but are not limited to, toluene-2,6-diisocyanate, 1,6-hexamethylene diisocyanate, 4,4'-diphenyl methane diisocyanate, 2,4'-diphenyl methane diisocyanate, 1,3-phenylene diisocyanate, 1,4-phenylene diisocyanate, toluene-2,4-diisocyanate, and combinations of two or more of the foregoing diisocyanate compounds.

Epoxide-containing polymer as used herein are polymers having the structure of Formula (XXVI) 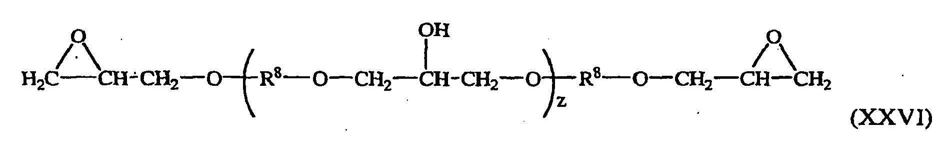 wherein R⁸ is a divalent functionality derived from a dihydroxy compound; wherein at least one of R⁸ is derived from a dihydroxy aromatic compound of Formula (I); and wherein "z" is 2 to 200.

The polymers described above may be a homopolymer containing structural units derived from a single dihydroxy aromatic compound represented by Formula (I) or a copolymer comprising structural units derived from two or more of the dihydroxy aromatic compound represented by Formula (I) or may be a copolymer comprising structural units derived from one or more dihydroxy aromatic compound represented by Formula (I) and structural units derived from other dihydroxy compounds. Accordingly, in one embodiment the polymer may comprise 1 mole percent to 100 mole percent of units derived from a dihydroxy aromatic compound of Formula (I). Within this range the amount may be greater than or equal to 5 mole percent, or, more specifically, greater than or equal to 10 mole percent. Also within this range the amount may be less than or equal to 80 mole percent, or, more specifically less than or equal to 50 mole percent.

In one embodiment the dihydroxy compounds that may be useful in forming the copolymer with the dihydroxy aromatic compound of Formula (I) may be represented by Formula (XXVII)

HO-R⁹-OH (XXVII),

wherein R⁹ includes a functionality of Formula (XX),

-A¹-Y¹-A²-; (XX)

and wherein Y¹, A¹ and A² are as defined above. In another embodiment the dihydroxy compound includes bisphenol compounds of general Formula (XXVIII): 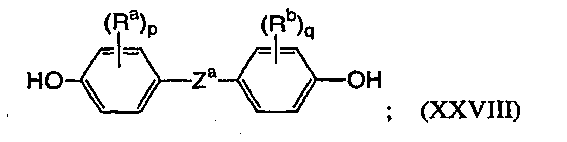 wherein R^{a} and R^{b} each represent a halogen atom or an aliphatic functionality having 1 to 10 carbon atoms and may be the same or different; p and q are each independently integers of 0 to 4; and Z^{a} represents one of the groups of Formula (XXIX): 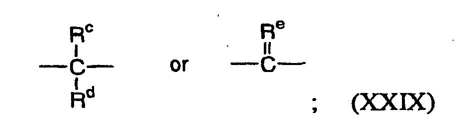 wherein R^{c} and R^{d} each independently represent a hydrogen atom or an aliphatic functionality having 1 to 10 carbon atoms or a cycloaliphatic functionality having 3 to 10 carbon atoms and R^{e} is a divalent aliphatic functionality having 1 to 10 carbon atoms.

Some illustrative, non-limiting examples of suitable dihydroxy compounds that may be used in combination with the dihydroxy aromatic compound of Formula (I) include, but are not limited to the following: resorcinol, 4-bromoresorcinol, hydroquinone, methyl hydroquinone, 1,1-bis-(4-hydroxy-3-methylphenyl)cyclohexane, 2-phenyl-3,3-bis(4-hydroxyphenyl)phthalimidine, eugenol siloxane bisphenol, 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1-naphthylmethane, 1,2-bis(4-hydroxyphenyl)ethane, 1,1-bis(4-hydroxyphenyl)-1-phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis (hydroxyphenyl)cyclopentane, 1,1-bis(4-hydroxyphenyl)cyclohexane, 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantine, (alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'-tetramethylspiro(bis)indane ("spirobiindane bisphenol"), 3,3-bis(4-hydroxyphenyl)phthalide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, and 2,7-dihydroxycarbazole, as well as combinations comprising at least one of the foregoing dihydroxy compounds.

Specific examples of the types of bisphenol compounds may include, but are not limited to 1,1-bis(4-hydroxyphenyl) methane, 1,1-bis(4-hydroxyphenyl) ethane, 2,2-bis (4-hydroxyphenyl) propane (bisphenol A), 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4-hydroxy-1-methylphenyl) propane, and 1,1-bis(4-hydroxy-t-butylphenyl) propane. Combinations comprising two or more of the foregoing dihydroxy compounds may also be used. In one embodiment the bisphenol compound employed is bisphenol A.

The polymer may be a branched polymer. Branched polymers comprising structural units derived from the dihydroxy aromatic compound of Formula (I) may be prepared by adding a branching agent during polymerization. These branching agents include polyfunctional organic compounds containing at least three functional groups selected from hydroxyl, carboxyl, carboxylic anhydride, haloformyl, and mixtures of the foregoing functional groups. Specific examples include, but are not limited to trimellitic acid, trimellitic anhydride, trimellitic trichloride, tris-p-hydroxy phenyl ethane, isatin-bis-phenol, tris-phenol TC (1,3,5-tris((p-hydroxyphenyl)isopropyl)benzene), tris-phenol-PA (4(4(1,1-bis(p-hydroxy-phenyl)-ethyl) alpha, alpha-dimethyl benzyl)phenol), 4-chloroformyl phthalic anhydride, trimesic acid, and benzophenone tetracarboxylic acid. All types of polycarbonate end groups are contemplated as being useful in the polycarbonate composition, provided that such end groups do not significantly affect desired properties of the thermoplastic compositions.

In one specific embodiment, the polymer is a polycarbonate wherein the polycarbonate is a linear homopolymer derived from dihydroxy aromatic compounds of Formula (I) or a copolymer comprising repeating units derived from dihydroxy aromatic compounds of Formula (I) and repeating units derived from bisphenol A. In one embodiment the polycarbonate may have a Tg greater than or equal to 140°C, or, more specifically, greater than or equal to 150°C, or, even more specifically greater than or equal to 160°C. Polycarbonates having a high Tg are also contemplated. The polycarbonate may have a weight average molecular weight of 10,000 to 200,000, or, more specifically, 20,000 to 100,000 as measured by gel permeation chromatography based on polystyrene standards.

Polycarbonates, polyesters and copolyester-carbonates may be manufactured by processes such as interfacial polymerization and melt polymerization. Although the reaction conditions for interfacial polymerization may vary, an exemplary process generally involves dissolving or dispersing a dihydroxy compound in aqueous sodium hydroxide or potassium hydroxide, adding the resulting mixture to a suitable water-immiscible solvent medium, and contacting the reactants with a carbonate precursor in the presence of a suitable catalyst such as triethylamine or a phase transfer catalyst, under controlled pH conditions, for example, 8 to 10. The most commonly used water immiscible solvents include, but are not limited to methylene chloride, 1,2-dichloroethane, chlorobenzene and toluene. Suitable carbonate precursors include, for example, a carbonyl halide such as carbonyl bromide or carbonyl chloride, or a haloformate such as a bishaloformate of a dihydric phenol (for example, the bischloroformates of bisphenol A, hydroquinone, or the like) or a glycol (for example, the bishaloformate of ethylene glycol, neopentyl glycol, polyethylene glycol, or the like) or esters (for example, bismethylsalicylate (bMSC)) or diphenyl carbonate (DPC). Combinations comprising at least one of the foregoing types of carbonate precursors may also be used. The resultant polymers, may have a weight average molecular weight (Mw) of 10,000 to 200,000, or, more specifically, 20,000 to 100,000 as measured by gel permeation chromatography based on polystyrene standards.

A chain stopper (also referred to as a capping agent) may be included during polymerization. The chain-stopper limits molecular weight growth rate, and so controls molecular weight in the polycarbonate. A chain-stopper may be at least one of mono-phenolic compounds, mono-carboxylic acid chlorides, and mono-chloroformates.

For example, mono-phenolic compounds suitable as chain stoppers include monocyclic phenols, such as phenol, C₁-C₂₂ alkyl-substituted phenols, p-cumylphenol, p-tertiary-butyl phenol, hydroxy diphenyl; and monoethers of diphenols, such as p-methoxyphenol. Alkyl-substituted phenols include those with branched chain alkyl substituents having 8 to 9 carbon atoms. A mono-phenolic UV absorber may be used as capping agent. Such compounds include 4-substituted-2-hydroxybenzophenones and their derivatives, aryl salicylates, monoesters of diphenols such as resorcinol monobenzoate, 2-(2-hydroxyaryl)-benzotriazoles and their derivatives, 2-(2-hydroxyaryl)-1,3,5-triazines and their derivatives. Specifically, mono-phenolic chain-stoppers include phenol, p-cumylphenol, and resorcinol monobenzoate.

Mono-carboxylic acid chlorides may also be suitable as chain stoppers. These include monocyclic, mono-carboxylic acid chlorides such as benzoyl chloride, C₁-C₂₂ alkyl-substituted benzoyl chloride, toluoyl chloride, halogen-substituted benzoyl chloride, bromobenzoyl chloride, cinnamoyl chloride, 4-nadimidobenzoyl chloride, and mixtures thereof; mono-carboxylic acid chlorides such as trimellitic anhydride chloride, and naphthoyl chloride; and mixtures of monocyclic and mono-carboxylic acid chlorides. Chlorides of aliphatic monocarboxylic acids with up to 22 carbon atoms are suitable. Functionalized chlorides of aliphatic monocarboxylic acids, such as acryloyl chloride and methacryoyl chloride, are also suitable. Also suitable are mono-chloroformates including monocyclic, mono-chloroformates, such as phenyl chloroformate, alkyl-substituted phenyl chloroformate, p-cumyl phenyl chloroformate, toluene chloroformate, and mixtures thereof.

Among the phase transfer catalysts that may be used are catalysts of the Formula (R^{g})₄Y⁺X⁵, wherein each R^{g} is the same or different, and is an alkyl group having 1 to 10 carbon atoms; Y is a nitrogen or phosphorus atom; and X⁵ is a halogen atom or an aliphatic functionality having 1 to 8 carbon atoms or aromatic functionality having 6 to 188 carbon atoms. Suitable phase transfer catalysts include, for example, [CH₃(CH₂)₃]₄NX⁵, [CH₃(CH₂)₃]₄PX⁵, [CH₃(CH₂)₅]₄NX⁵, [CH₃(CH₂)₆]₄NX⁵, [CH₃(CH₂)₄]₄NX⁵, CH₃[CH₃(CH₂)₃]₃NX⁵, and CH₃[CH₃(CH₂)₂]₃NX⁵, wherein X⁵ is chloride, bromide, an aliphatic functionality having 1 to 8 carbon atoms or aromatic functionality having 6 to 188 carbon atoms. An effective amount of a phase transfer catalyst may be 0.1 to 10 wt. percent based on the weight of bisphenol in the phosgenation mixture. In another embodiment an effective amount of phase transfer catalyst may be 0.5 to 2 wt. percent based on the weight of bisphenol in the phosgenation mixture.

Alternatively, melt processes may be used to make the polycarbonates. Generally, in the melt polymerization process, polycarbonates may be prepared by co-reacting, in a molten state, the dihydroxy reactant(s) and a diaryl carbonate ester, such as diphenyl carbonate, bis methyl salicylate or a combination thereof, in the presence of a transesterification catalyst in a Banbury® mixer, twin screw extruder, or the like to form a uniform dispersion. Volatile monohydric phenol is removed from the molten reactants by distillation and the polymer is isolated as a molten residue.

The transesterifcation catalysts capable of effecting reaction between the ester and the dihydroxy compound may comprise a single compound or a mixture of compounds and may be employed in combination with one or more co-catalysts such as quaternary ammonium salts or quaternary phosphonium salts. Suitable transesterification catalysts include, but are not limited to, alkali metal hydroxides, for example, lithium hydroxide, sodium hydroxide, potassium hydroxide, and mixtures thereof; alkaline earth metal hydroxides, for example, calcium hydroxide, barium hydroxide, and mixtures thereof; alkali metal salts of carboxylic acids, for example, lithium acetate, sodium benzoate, and dipotassium dodecanedioate; alkaline earth metal salts of carboxylic acids, for example, calcium benzoate, calcium adipate, and barium acetate; salts of a polycarboxylic acid, for example, tetrasodium ethylenediamine tetracarboxylate and disodium magnesium ethylenediamine tetracarboxylate and salts of non- volatile acids, for example, alkaline earth metal salts of phosphates, alkali metal salts of phosphates, alkaline earth metal salts of phosphates, alkali metal salts of sulfates, alkaline earth metal salts of sulfates, alkali metal salts of metal oxo acids, and alkaline earth metal salts of metal oxo acids. Specific examples of salts of non-volatile acids include NaH₂PO₃, NaH₂PO₄, Na₂H₂PO₃, KH₂PO₄, CsH₂PO₄, CsH₂PO₄, Cs₂H₂PO₄, Na₂SO₄, NaHSO₄, NaSbO₃, LiSbO₃, KSbO₃, Mg(SbO₃)₂, Na₂GeO₃, K₂GeO₃, Li₂GeO₃, MgGeO₃, Mg₂GeO₄, and mixtures thereof. As used herein the term "non-volatile acid" means that the acid from which the catalyst is made has no appreciable vapor pressure under melt polymerization conditions. Examples of non-volatile acids include phosphorous acid, phosphoric acid, sulfuric acid, and metal "oxo acids" such as the oxo acids of germanium, antimony, niobium and the like.

As mentioned, melt polymerization may be practiced using a co-catalyst. Typically, the co-catalyst is a quaternary ammonium salt or quaternary phosphonium salt and is used in an amount corresponding to 10 to 250 times the molar amount of melt polymerization catalyst used. The catalyst and co-catalyst, may be added to the reaction mixture either simultaneously, or the catalyst and co-catalyst may be added separately at different stages of the polymerization reaction.

When activated carbonate precursors (i.e., carbonate precursors that react faster than diphenyl carbonate) such as bMSC are used to make the polycarbonate, polyester and copolyester-carbonate polymers described herein the polymers can comprise certain physical differences compared to similar polymers prepared using other melt or interfacial methods. For example, such polymers typically contain some type of internal methyl salicylate "kink" structures such as shown below, and a certain amount of endcap structures indicative of the use of bMSC as shown in units represented by Formula (XVI), Formula (XVII) and Formula (XVIII)

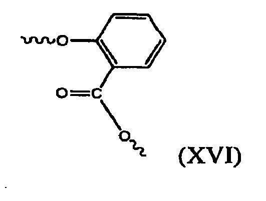

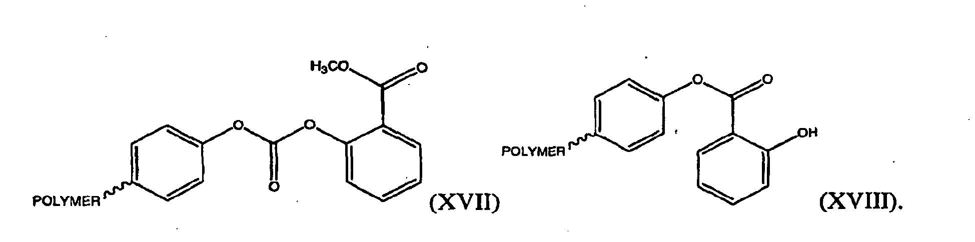

The copolyester-polycarbonate resins may also be prepared by interfacial polymerization. Rather than utilizing the dicarboxylic acid per se, it is possible to employ the reactive derivatives of the acid, such as the corresponding acid halides, in particular the acid dichlorides and the acid dibromides. Thus, for example instead of using isophthalic acid, terephthalic acid, or mixtures thereof, it is possible to employ isophthaloyl dichloride, terephthaloyl dichloride, and mixtures thereof.

The polyurethanes may be prepared by reacting a dihydroxy aromatic compound of Formula (I) with a diisocyanate compound having Formula (XXV)

Q(NCO)₂ (XXV)

wherein Q comprises a divalent aliphatic functionality having 2 to 28 carbons, a divalent cycloaliphatic functionality having 4 to 15 carbons, or a divalent aromatic functionality having 6 to 15 carbons. This reaction can be carried out by methods known in the art.

The epoxide containing polymers can be prepared by reacting a dihydroxy aromatic compound of Formula (I) with epichlorohydrin to form a diglycidyl ether compound of Formula (XXX) 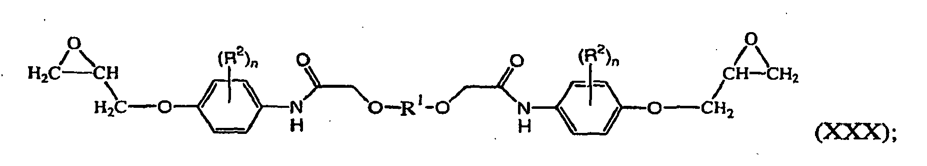 and polymerizing the diglycidyl ether compound of Formula (XXX) to provide the epoxide-containing polymer having Formula (XVI). This reaction can be carried out by methods known in the art.

In addition to the polymers described above, it is also possible to use combinations of the polymer with other thermoplastic polymers, for example combinations of polycarbonates and/or polycarbonate copolymers with polyamides, polyesters, other polycarbonates; copolyester-polycarbonates, olefin polymers such as acrylonitrile butadiene styrene, polystyrene, polyethylene; polysiloxanes, polysilanes and polysulfones. As used herein, a "combination" is inclusive of all mixtures, blends and alloys. In certain embodiments the one or more additional resins may be present preferably in an amount less than or equal to 40 weight percent, more preferably less than or equal to 35 weight percent and most preferably less than or equal to 30 weight percent based on the total weight of the polymer composition.

In addition to the polycarbonate resin, the thermoplastic composition may include various additives ordinarily incorporated in resin compositions of this type, with the proviso that the additives are preferably selected so as to not significantly adversely affect the desired properties of the thermoplastic composition. Mixtures of additives may be used. Such additives may be mixed at a suitable time during the mixing of the components for forming the composition.

Exemplary additives include such materials as fillers or reinforcing agents, thermal stabilizers, radiation stabilizers, antioxidants, light stabilizers, UV stabilizers, plasticizers, visual effect enhancers, extenders, antistatic agents, catalyst quenchers, mold release agents, flame retardants, infrared shielding agents, whitening agents, blowing agents, anti-drip agents, impact modifiers and processing aids. The different additives that can be incorporated in the polymer compositions of the present invention are typically commonly used and known to those skilled in the art.

Suitable fillers or reinforcing agents include, for example, silicates and silica powders such as aluminum silicate (mullite), synthetic calcium silicate, zirconium silicate, fused silica, crystalline silica graphite, natural silica sand, or the like; boron powders such as boron-nitride powder, boron-silicate powders, or the like; oxides such as TiO2, aluminum oxide, magnesium oxide, or the like; calcium sulfate (as its anhydride, dihydrate or trihydrate); calcium carbonates such as chalk, limestone, marble, synthetic precipitated calcium carbonates, or the like; talc, including fibrous, modular, needle shaped, lamellar talc, or the like; wollastonite; surface-treated wollastonite; glass spheres such as hollow and solid glass spheres, silicate spheres, cenospheres, aluminosilicate (armospheres), or the like; kaolin, including hard kaolin, soft kaolin, calcined kaolin, kaolin comprising various coatings known in the art to facilitate compatibility with the polymeric matrix resin, or the like; single crystal fibers or "whiskers" such as silicon carbide, alumina, boron carbide, iron, nickel, copper, or the like; fibers (including continuous and chopped fibers) such as asbestos, carbon fibers, glass fibers, such as E, A, C, ECR, R, S, D, or NE glasses , or the like; sulfides such as molybdenum sulfide, zinc sulfide or the like; barium compounds such as barium titanate, barium ferrite, barium sulfate, heavy spar, or the like; metals and metal oxides such as particulate or fibrous aluminum, bronze, zinc, copper and nickel or the like; flaked fillers such as glass flakes, flaked silicon carbide, aluminum diboride, aluminum flakes, steel flakes or the like; fibrous fillers, for example short inorganic fibers such as those derived from blends comprising at least one of aluminum silicates, aluminum oxides, magnesium oxides, and calcium sulfate hemihydrate or the like; natural fillers and reinforcements, such as wood flour obtained by pulverizing wood, fibrous products such as cellulose, cotton, sisal, jute, starch, cork flour, lignin, ground nut shells, corn, rice grain husks or the like; organic fillers such as polytetrafluoroethylene; reinforcing organic fibrous fillers formed from organic polymers capable of forming fibers such as poly(ether ketone), polyimide, polybenzoxazole, poly(phenylene sulfide), polyesters, polyethylene, aromatic polyamides, aromatic polyimides, polyetherimides, polytetrafluoroethylene, acrylic resins, poly(vinyl alcohol) or the like; as well as additional fillers and reinforcing agents such as mica, clay, feldspar, flue dust, fillite, quartz, quartzite, perlite, tripoli, diatomaceous earth, carbon black, or the like, or combinations comprising at least one of the foregoing fillers or reinforcing agents.

The fillers and reinforcing agents may be coated with a layer of metallic material to facilitate conductivity, or surface treated with silanes to improve adhesion and dispersion with the polymeric matrix resin. In addition, the reinforcing fillers may be provided in the form of monofilament or multifilament fibers and may be used either alone or in combination with other types of fiber, through, for example, co-weaving or core/sheath, side-by-side, orange-type or matrix and fibril constructions, or by other methods known to one skilled in the art of fiber manufacture. Suitable cowoven structures include, for example, glass fiber-carbon fiber, carbon fiber-aromatic polyimide (aramid) fiber, and aromatic polyimide fiberglass fiber or the like. Fibrous fillers may be supplied in the form of, for example, rovings, woven fibrous reinforcements, such as 0-90 degree fabrics or the like; non-woven fibrous reinforcements such as continuous strand mat, chopped strand mat, tissues, papers and felts or the like; or three-dimensional reinforcements such as braids.

Suitable thermal stabilizer additives include, for example, organophosphites such as triphenyl phosphite, tris-(2,6-dimethylphenyl)phosphite, tris-(mixed mono-and dinonylphenyl)phosphite or the like; phosphonates such as dimethylbenzene phosphonate or the like, phosphates such as trimethyl phosphate, or the like, or combinations comprising at least one of the foregoing heat stabilizers.

Non-limiting examples of antioxidants that can be used in the polymer compositions of the present invention include tris(2, 4-di-tert-butylphenyl)phosphite; 3,9-di(2, 4-di-tert-butylphenoxy)-2,4,8,10-tetraoxa-3,9- diphosphaspiro[5.5]undecane; 3,9-di(2,4-dicumylphenoxy)-2,4,8,10-tetraoxa-3,9-diphosphaspiro[5.5]undecane; tris(p-nonylphenyl)phosphite; 2,2',2"- nitrilo[triethyl-tris[3,3',5,5'-tetra-tertbutyl-1,1'-biphenyl-2'- diyl]phosphite]; 3, 9-distearyloxy-2,4,8,10-tetraoxa-3,9-diphosphaspiro[5. 5]undecane; dilauryl phosphite; 3,9-di[2,6-di-tert-butyl-4-methylphenoxy]-2, 4, 8, 10-tetraoxa-3,9-diphosphaspiro[5.5]undecane; tetrakis(2, 4-di-tert-butylphenyl)-4, 4'-bis(diphenylene)phosphonite; distearyl pentaerythritol diphosphite; diisodecyl pentaerythritol diphosphite; 2, 4, 6-tri-tert-butylphenyl-2-butyl-2-ethyl-1,3-propanediol phosphite; tristearyl sorbitol triphosphite; tetrakis(2, 4-di-tert-butylphenyl)-4,4'- biphenylene diphosphonite; (2, 4, 6-tri-tert-butylphenyl)-2-butyl-2-ethyl-1, 3-propanediolphosphite; triisodecylphosphite; and mixtures of phosphites containing at least one of the foregoing.

Non-limiting examples of UV stabilizers that can be used include 2-(2'-hydroxyphenyl)-benzotriazoles, for example, the 5'-methyl-; 3',5'-di- tert.-butyl-; 5'-tert.-butyl-; 5'-(1,1,3,3-tetramethylbutyl)-; 5-chloro- 3',5'-di-tert.-butyl-; 5-chloro-3'-tert.-butyl-5'-methyl-; 3'-sec.-butyl- 5'-tert.-butyl-; 3'-alpha -methylbenzyl -5'-methyl; 3'- alpha- methylbenzyl-5'-methyl-5-chloro-; 4'-hydroxy-; 4'-methoxy-; 4'-octoxy-; 3',5'-di-tert.-amyl-; 3'-rnethyl-5'-carbomethoxyethyl-; 5-chloro-3',5'- di-tert.-amyl-derivatives; and Tinuvin® 234 (available from Ciba Specialty Chemicals). Also suitable are the 2, 4-bis-(2'-hydroxyphenyl)-6-alkyl-s-triazines, for example, the 6-ethyl-; 6- heptadecyl- or 6-undecyl-derrvatives. 2-Hydroxybenzophenones for example, the 4-hydroxy-; 4-methoxy-; 4-octoxy-; 4- decyloxy-; 4-dodecyloxy-; 4-benzyloxy-; 4,2',4'-trihydroxy-; 2,2',4,4'- tetrahydroxy- or 2'-hydroxy-4,4'-dimethoxy-derivative. 1,3-bis-(2'-Hydroxybenzoyl)-benzenes, for example, 1,3-bis-(2'-hydroxy-4'- hexyloxy-benzoyl)-benzene; 1,3-bis-(2'-hydroxy-4'-octyloxy-benzoyl)- benzene or 1,3-bis-(2'-hydroxy-4'-dodecyloxybenzoyl)-benzene may also be employed. Esters of optionally substituted benzoic acids, for example, phenylsalicylate; octylphenylsalicylate; dibenzoylresorcin; bis-(4-tert.-butylbenzoyl)- resorcin; benzoylresorcin; 3,5-di-tert.-butyl-4-hydroxybenzoic acid-2,4- di-tert.-butylphenyl ester or -octadecyl ester or -2-methyl-4,6-di-tert.- butyl ester may likewise be employed. Acrylates, for example, alpha -cyano- beta, beta -diphenylacrylic acid-ethyl ester or isooctyl ester, alpha -carbomethoxy-cinnamic acid methyl ester, alpha-cyano-beta-methyl-p-methoxy-cinnamic acid methyl ester or -butyl ester or N(beta-carbomethoxyvinyl)-2-methyl-indoline may likewise be employed. Oxalic acid diamides, for example, 4,4'-di-octyloxy-oxanilide; 2,2'-di- octyloxy-5,5'-di-tert.-butyl-oxanilide; 2,2'-di-dodecyloxy-5,5-di-tert.- butyl-oxanilide; 2-ethoxy-2'-ethyl-oxanilide; N,N'-bis-(3-dimethyl- aminopropyl)-oxalamide; 2-ethoxy-5-tert.-butyl-2'-ethyloxanilide and the mixture thereof with 2-ethoxy-2'-ethyl-5,4'-di-tert.-butyl-oxanilide; or mixtures of ortho- and para-methoxy- as well as of o- and p-ethoxy-disubstituted oxanilides are also suitable as UV stabilizers. Preferably the ultraviolet light absorber used in the instant compositions is 2-(2-hydroxy-5-methylphenyl)-2H-benzotriazole; 2-(2- hydroxy-3,5-di-tert-amylphenyl)-2H-benzotriazole; 2-[2-hydroxy-3,5-di- (alpha,alpha-dimethylbenzyl)phenyl]-2H-benzotriazole; 2-(2-hydroxy-5-tert- octylphenyl)-2H-benzotriazole; 2-hydroxy-4-octyloxybenzophenone; nickel bis(O-ethyl 3,5-di-tert-butyl-4-hydroxybenzylphosphonate); 2,4-dihydroxybenzophenone; 2-(2-hydroxy-3-tert-butyl-5-methylphenyl)-2H-benzotriazole; nickel butylamine complex with 2,2'-thiobis(4-tert- butylphenol); 2-ethoxy-2'-ethyloxanilide; 2-ethoxy-2'-ethyl-5,5'-ditert- butyloxanilide or a mixture thereof.

Plasticizers, lubricants, and/or mold release agents additives may also be used. There is considerable overlap among these types of materials, which include, for example, phthalic acid esters such as dioctyl-4,5-epoxy-hexahydrophthalate; tris-(octoxycarbonylethyl)isocyanurate; tristearin; di- or polyfunctional aromatic phosphates such as resorcinol tetraphenyl diphosphate (RDP), the bis(diphenyl) phosphate of hydroquinone and the bis(diphenyl) phosphate of bisphenol-A; poly-alpha-olefins; epoxidized soybean oil; silicones, including silicone oils; esters, for example, fatty acid esters such as alkyl stearyl esters, for example, methyl stearate; stearyl stearate and pentaerythritol tetrastearate. Mixtures of methyl stearate and hydrophilic and hydrophobic nonionic surfactants comprising polyethylene glycol polymers, polypropylene glycol polymers, and copolymers thereof, for example, methyl stearate and polyethylene-polypropylene glycol copolymers in a suitable solvent; waxes such as beeswax, montan wax, paraffin wax or the like.

Visual effect enhancers, sometimes known as visual effects additives or pigments may be present in an encapsulated form, a non-encapsulated form, or laminated to a particle comprising polymeric resin. Some non-limiting examples of visual effects additives are aluminum, gold, silver, copper, nickel, titanium, stainless steel, nickel sulfide, cobalt sulfide, manganese sulfide, metal oxides, white mica, black mica, pearl mica, synthetic mica, mica coated with titanium dioxide, metal-coated glass flakes, and colorants, including but not limited, to Perylene Red. The visual effect additive may have a high or low aspect ratio and may comprise greater than 1 facet. Dyes may be employed such as Solvent Blue 35, Solvent Blue 36, Disperse Violet 26, Solvent Green 3, Anaplast Orange LFP, Perylene Red, and Morplas Red 36. Fluorescent dyes may also be employed including, but not limited to, Permanent Pink R (Color Index Pigment Red 181, from Clariant Corporation), Hostasol Red 5B (Color Index #73300, CAS # 522-75-8, from Clariant Corporation) and Macrolex Fluorescent Yellow 10GN (Color Index Solvent Yellow 160:1, from Bayer Corporation). Pigments such as titanium dioxide, zinc sulfide, carbon black, cobalt chromate, cobalt titanate, cadmium sulfides, iron oxide, sodium aluminum sulfosilicate, sodium sulfosilicate, chrome antimony titanium rutile, nickel antimony titanium rutile, and zinc oxide may be employed. Visual effect additives in encapsulated form usually comprise a visual effect material such as a high aspect ratio material like aluminum flakes encapsulated by a polymer. The encapsulated visual effect additive has the shape of a bead.

The term "antistatic agent" refers to monomeric, oligomeric, or polymeric materials that can be processed into polymer resins and/or sprayed onto materials or articles to improve conductive properties and overall physical performance. Examples of monomeric antistatic agents include glycerol monostearate, glycerol distearate, glycerol tristearate, ethoxylated amines, primary, secondary and tertiary amines, ethoxylated alcohols, alkyl sulfates, alkylarylsulfates, alkylphosphates, alkylaminesulfates, alkyl sulfonate salts such as sodium stearyl sulfonate, sodium dodecylbenzenesulfonate or the like, quaternary ammonium salts, quaternary ammonium resins, imidazoline derivatives, sorbitan esters, ethanolamides, betaines, or the like, or combinations comprising at least one of the foregoing monomeric antistatic agents.

Exemplary polymeric antistatic agents include certain polyesteramides, polyether-polyamide (polyetheramide) block copolymers, polyetheresteramide block copolymers, polyetheresters, or polyurethanes, each containing polyalkylene oxide units that may be polyalkylene glycol functionality, for example, polyethylene glycol, polypropylene glycol and polytetramethylene glycol. Such polymeric antistatic agents are commercially available, such as, for example, Pelestat^{™} 6321 (Sanyo), Pebax^{™} H1657 (Atofina), and Irgastat^{™} P18 and P22 (Ciba-Geigy). Other polymeric materials that may be used as antistatic agents are inherently conducting polymers such as polyaniline (commercially available as PANIPOL®EB from Panipol), polypyrrole and polythiophene (commercially available from Bayer), which retain some of their intrinsic conductivity after melt processing at elevated temperatures. In one embodiment, carbon fibers, carbon nanofibers, carbon nanotubes, carbon black, or any combination of the foregoing may be used in a polymeric resin containing chemical antistatic agents to render the composition electrostatically dissipative.

Non-limiting examples of mold release compositions include esters of long-chain aliphatic acids and alcohols such as pentaerythritol, guerbet alcohols, long-chain ketones, siloxanes, alpha.-olefin polymers, long-chain alkanes and hydrocarbons having 15 to 600 carbon atoms.

Non-limiting examples of flame retardants that can be used include potassium diphenylsulfone sulfonate, perfluoroalkane sulfonates and phosphite esters of polyhydric phenols, such as resorcinol and bisphenol A.

The thermoplastic composition may optionally comprise an impact modifier. The impact modifier resin added to the thermoplastic composition in an amount corresponding to 1 percent to 30 percent by weight, based on the total weight of the composition. Suitable impact modifiers include those comprising one of several different rubbery modifiers such as graft or core shell rubbers or combinations of two or more of these modifiers. Impact modifiers are illustrated by acrylic rubber, ASA rubber, diene rubber, organosiloxane rubber, ethylene propylene diene monomer (EPDM) rubber, styrene-butadiene-styrene (SBS) rubber; styrene-ethylene-butadiene-styrene (SEBS) rubber, acrylonitrile-butadiene-styrene (ABS) rubber, methacrylate-butadiene-styrene (MBS) rubber, styrene acrylonitrile copolymer and glycidyl ester impact modifier.

Non-limiting examples of processing aids that can be used include Doverlube® FL-599 (available from Dover Chemical Corporation), Polyoxyter® (available from Polychem Alloy Inc.), Glycolube® P (available from Lonza Chemical Company), pentaerythritol tetrastearate, Metablen® A-3000 (available from Mitsubishi Rayon) and neopentyl glycol dibenzoate.

Radiation stabilizers may also be present in the thermoplastic composition, specifically gamma-radiation stabilizers. Suitable gamma-radiation stabilizers include diols, such as ethylene glycol, propylene glycol, 1,3-propanediol, 1,2-butanediol, 1,4-butanediol, meso-2,3-butanediol, 1,2-pentanediol, 2,3-pentanediol, 1,4-pentanediol and 1,4-hexandiol.; alicyclic alcohols such as 1,2-cyclopentanediol and 1,2-cyclohexanediol; branched acyclic diols such as 2,3-dimethyl-2,3-butanediol (pinacol), and polyols, as well as alkoxy-substituted cyclic or acyclic alkanes. Alkenols, with sites of unsaturation, are also a useful class of alcohols, examples of which include 4-methyl-4-penten-2-ol, 3-methyl-pentene-3-ol, 2-methyl-4-penten-2-ol, 2,4-dimethyl-4-pene-2-ol, and 9-decen-1-ol. Another class of suitable alcohols is the tertiary alcohols, which have at least one hydroxy substituted tertiary carbon. Examples of these include 2-methyl-2,4-pentanediol (hexylene glycol), 2-phenyl-2-butanol, 3-hydroxy-3-methyl-2-butanone and 2-phenyl-2-butanol., and cycoloaliphatic tertiary carbons such as 1-hydroxy-1-methyl-cyclohexane. Another class of suitable alcohols is hydroxymethyl aromatics, which have hydroxy substitution on a saturated carbon attached to an unsaturated carbon in an aromatic ring. The hydroxy substituted saturated carbon may be a methylol group (-CH₂OH) or it may be a member of a more complex hydrocarbon group such as would be the case with (-CR¹²HOH) or (-CR₂¹²OH) wherein R¹² is a complex or a simply hydrocarbon. Specific hydroxy methyl aromatics may be benzhydrol, 1,3-benzenedimethanol, benzyl alcohol, 4-benzyloxy benzyl alcohol and benzyl benzyl alcohol. Specific alcohols are 2-methyl-2,4-pentanediol (also known as hexylene glycol), polyethylene glycol, polypropylene glycol.

Where a foam is desired, a blowing agent may be added to the composition. Suitable blowing agents include for example, low boiling halohydrocarbons; those that generate carbon dioxide; blowing agents that are solid at room temperature and that when heated to temperatures higher than their decomposition temperature, generate gases such as nitrogen, carbon dioxide, ammonia gas or the like, such as azodicarbonamide, metal salts of azodicarbonamide, 4,4' oxybis(benzenesulfonylhydrazide), sodium bicarbonate, ammonium carbonate, or the like, or combinations comprising at least one of the foregoing blowing agents.

Anti-drip agents may also be used, for example a fibril forming or non-fibril forming fluoropolymer such as polytetrafluoroethylene (PTFE). The anti-drip agent may be encapsulated by a rigid copolymer as described above, for example styreneacrylonitrile copolymer (SAN). PTFE encapsulated in SAN is known as TSAN. Encapsulated fluoropolymers may be made by polymerizing the encapsulating polymer in the presence of the fluoropolymer, for example an aqueous dispersion. TSAN may provide significant advantages over PTFE, in that TSAN may be more readily dispersed in the composition. A suitable TSAN may comprise, for example, 50 wt. percent PTFE and 50 wt. percent SAN, based on the total weight of the encapsulated fluoropolymer. The SAN may comprise, for example, 75 wt. percent styrene and 25 wt. percent acrylonitrile based on the total weight of the copolymer. Alternatively, the fluoropolymer may be pre-blended in some manner with a second polymer, such as for, example, an aromatic polycarbonate resin or SAN to form an agglomerated material for use as an anti-drip agent. Either method may be used to produce an encapsulated fluoropolymer.

The thermoplastic compositions may be manufactured by methods generally available in the art, for example, in one embodiment, in one manner of proceeding, powdered polymer resin and/or other optional components are first blended, in a Henschel^{™} high speed mixer. Other low shear processes including but not limited to hand mixing may also accomplish this blending. The blend is then fed into the throat of a twinscrew extruder via a hopper. Alternatively, one or more of the components may be incorporated into the composition by feeding directly into the extruder at the throat and/or downstream through a sidestuffer. Such additives may also be compounded into a master-batch with a desired polymeric resin and fed into the extruder. The extruder is generally operated at a temperature higher than that necessary to cause the composition to flow. The extrudate is immediately quenched in a water batch and pelletized. The pellets, so prepared, when cutting the extrudate may be one-fourth inch long or less as desired. Such pellets may be used for subsequent molding, shaping, or forming.

Shaped, formed, or molded articles comprising the polycarbonate compositions are also provided. The polycarbonate compositions may be molded into useful shaped articles by a variety of means such as injection molding, extrusion, rotational molding, blow molding and thermoforming to form articles such as, for example, computer and business machine housings such as housings for monitors, handheld electronic device housings such as housings for cell phones, electrical connectors, and components of lighting fixtures, ornaments, home appliances, roofs, greenhouses, sun rooms, swimming pool enclosures and automotive application. (e.g., forward lighting enclosures for car headlamps).

The disclosure is explained in more detail with reference to the following non-limiting Examples.

### EXAMPLES

High Performance Liquid Chromatography (HPLC) was used to identity the conversion of product compound. An Xterra C18 column, length 15 centimeters, inner diameter 4.6 millimeters and thickness 5 micrometers was used for the analysis. The column temperature was maintained at 30°C. The column was eluted with 90% of water (containing 0.05% of orthophosphoric acid) and 10% acetonitrile. The flow rate of sample in the column was maintained at 1.00 ml/min and amount of sample injected was 5 microliter. The total run time was 35 min.

Proton NMR spectra for the starting materials and products described herein were measured using a 300 megahertz Bruker NMR spectrometer using deuterated chloroform or d₆-dimethylsulfoxide as a solvent. Compounds were further characterized by a liquid chromatograph-mass spectrometer (LC-MS) system, comprising a liquid chromatograph and a Quattro Ultima Pt mass spectrometer.

### EXAMPLE 1

This example provides a method for the preparation of N-(4-Hydroxy-phenyl)-2-[4-[(4-hydroxy-phenylcarbamoyl)-methoxy]-phenoxy]-acetamide. The method includes 3 steps as described below.

### STEP A: Preparation of (4-carboxymethoxy-phenoxy)-acetic acid (diacid)

To an aqueous solution of sodium hydroxide (88 grams (g) in 267 milliliters (ml) of water) was charged 1,4-hydroquinone (55 g; purity greater than 99%) portion-wise and the resultant mixture was stirred under nitrogen atmosphere. The mixture was cooled to 10°C and an aqueous solution of chloroacetic acid (CAA, 94.5 g in 47.2 ml of water) was added dropwise to the hydroquinone solution prepared above, maintaining the temperature of the resultant reaction mixture below 10°C. The reaction mixture was stirred for 30 minutes (min) at 10°C. The reaction mixture was then heated to 80°C and maintained at 80°C for 36 hours (hrs) to 48 hrs, under N₂ atmosphere. The reaction was monitored by HPLC after 6 hrs after heating to 80°C. An aqueous solution of chloroacetic acid (9.4 g dissolved in 19.8 ml water) was added since the presence of monoacid was detected in the reaction mixture. Heating was continued at 80°C for an additional 1 to 3 hrs and the procedure repeated until the concentration of monoacid was less than 3% (based on LC/NMR). The reaction mixture was cooled to 25°C and the contents acidified by adding aqueous HCl (1:1; 0-5°C) with stirring. The solid was collected by filtration and washed with water until the washings were neutral. The solid was dried at 85°C in an oven for 8 hrs. The product weighed 45 g and had a purity of 90 percent as determined by HPLC.

Purification of (4-carboxymethoxy-phenoxy)-acetic acid (diacid):

The diacid prepared above (30 g) was dissolved in 90 ml of dimethylformamide and warmed to 60 °C and charcoal (0.5 g) was added. The hot mixture was filtered through a celite bed. The filtrate was diluted with 90 ml of methanol and 600 ml of demineralized water under stirring to precipitate the solid. The resulting solid was filtered and dried. The product obtained after purification weighed 32 g at 99 percent purity. The NMR peaks for the product were ¹H NMR: DMSO-d₆: δ 4.6 (m, 4H, O-CH₂), 6.85 (m, 4H, Ar-O-H). (DMSO - dimethylsulfoxide)

### STEP B: Preparation of diacid chloride of (4-carboxymethoxy-phenoxy)-acetic acid

The purified diacid from Step A (40.25 g, dry product) was charged to a reaction flask. 1,2-dichloroethane (125 ml) and dimethylformamide (5 ml) were then added to the diacid. Thionyl chloride (47.2 g) was added dropwise to the diacid-dichloroethane mixture. After completion of the addition of thionyl chloride, the resultant mixture was heated at 85°C and maintained for 6 hrs. Then the mixture was cooled to room temperature (25°C). The excess thionyl chloride and 1,2-dichloroethane were distilled off at 55 to 60 °C under vacuum. After complete removal of solvents and excess thionyl chloride, 1,2-dichloroethane (25 ml) was charged to the residue and distilled off to get the diacid chloride solids.

STEP C: N-(4-hydroxy-phenyl)-2-[4-[(4-hydroxy-phenylcarbamoyl)-methoxy]-phenoxy]-acetamide.

The diacid chloride obtained from Step B, was dissolved in dimethylformamide (100 ml) and stored under N₂ atmosphere. A solution of 4-amino phenol (26.2 g dissolved in 125 ml dimethylformamide) was put under a N₂ atmosphere. The mixture was stirred to get a clear solution and then triethylamine (25 g) was added. The mixture was cooled to 0 to 5°C and then stirred for 30 min. The diacid chloride solution prepared above was charged in a dropping funnel and added over a period of 30 minutes to the stirred solution of aminophenol-triethylamine kept at 0 to 5°C. The mixture was then stirred for 1 hr at 5°C and stirred at room temperature for 2 hrs. The reaction was monitored by HPLC to check for the disappearance of 4-aminophenol. When the aminophenol content was below 2%, the mixture was acidified with HCl (12 ml, 1:1, 0 to 5°C). The resultant mixture was stirred for 30 min and then the solid was filtered under suction. The solid was washed with water until the washings were neutral. The material was dried in an oven at 65 -to 70°C for 6 hrs. The product obtained weighed 45 g and showed 90 to 92 percent purity based on HPLC.

### Purification of the final product

The product obtained in step C (50 g) was dissolved in 200 ml of dimethylformamide and heated to obtain a clear solution (70°C). An aqueous solution of sodium bisulphate (0.5 g in 1 ml of water) was added to the dimethylformamide solution and stirred for 20 min. Charcoal (2 g) was added to the mixture and heated for 5 min. The resulting slurry was filtered. Water (200 ml) was added to the filtrate under stirring. The resulting solid was filtered and the material air dried under vacuum for 2 hrs. The solids were suspended in 300 ml of acetonitrile and heated to 65°C. The slurry was then filtered. The solid was dried and purity checked by HPLC. The solid was dried in an oven at 85°C for 6 hrs. The product obtained weighed 36 g wit purity of 99.2 % based on LC. NMR data : ¹H NMR: DMSO-d₆: δ 4.52 (m, 4H, O-CH2), 6.7 (m, 4H, Ar-O-H), 6.9 (m, 4H, Ar-NH-H), 7.4 (m, 4H, quinol-H), 9.3 (bs, 2H, OH), 9.7 (bs, 2H, NH)

### EXAMPLE 2

This example provides a method for the preparation of N-(4-hydroxy-phenyl)-2-[4-[(4-hydroxy-phenylcarbamoyl)-methoxy]-phenoxy]-acetamide. The method includes 3 steps as described below.

### STEP A: Preparation of (4-carboxymethoxy-2-methyl-phenoxy)-acetic acid (diacid)

(4-carboxymethoxy-2-methyl-phenoxy)-acetic acid was prepared using the same procedure described for Step A in Example 1 except that hydroquinone was substituted with methyl hydroquinone (62 g). Other reagents and solvents were used as described in the procedure mentioned above. The product obtained weighed 50 g with a purity of 95 percent as indicated by HPLC.

### Purification of the (4-carboxymethoxy-2-methyl-phenoxy)-acetic acid (diacid).

A solution of diacid (30 g in 90 ml of dimethylformamide) was warmed to 60°C and charcoal (0.5 g) was added. The slurry was filtered through celite bed and the filtrate diluted with methanol (90 ml) and demineralized water (600 ml) under stirring. The resulting solid was filtered and dried. The product obtained after purification weighed 36 g with a purity of 99 percent as indicated by LC. NMR data : ¹H NMR: DMSO-d₆: δ 2.18 (m, 3H), 4.6 (m, 4H, O-CH₂), 6.6-6.8 (m, 4H, Ar-O-H).

### STEP B: Preparation of (4-chlorocarbonylmethoxy-2-methyl-phenoxy)-acetyl chloride (diacid chloride)

The diacid chloride was prepared using the same procedure as used in Step B of Example 1 except that (4-carboxymethoxy-2-methyl-phenoxy)-acetic-acid of Example 2 was used in place of (4-carboxymethoxy-phenoxy)-acetic acid of Example 1.

### STEP C: Preparation of N-(4-hydroxy-phenyl)-2-{4-[(4-hydroxy-phenylcarbamoyl)-methoxy]-phenoxy} -acetamide

The acetamide was prepared in a similar manner as in STEP C of Example 1, except in that the diacid chloride of (4-carboxymethoxy-2-methyl-phenoxy)-acetic acid of Example 2 was used in place of (4-carboxymethoxy-phenoxy)-acetic acid of Example 1. The product obtained weighed 45 g with a purity of 90 to 92 percent as indicated by LC/MS. NMR Data : ¹H NMR: DMSO-d₆: δ 2.3 (s, 3H, Me-H), 4.6 (m, 4H, Ar-O-CH2), 6.6 (m, 7H, Ar-O-H and quinol-H), 7.4 (m, 4H, Ar-NH-H), 9.3 (bs, 2H, OH), 9.7 (bs, 2H, NH).

### EXAMPLE 3

This example provides a method for the preparation of N-(4-hydroxy-phenyl)-2-(4-(1-methyl-1-[4-(4-hydroxyphenlycarbomyl-methoxy)-phenyl]-ethyl)-phenoxy)-acetamide. The method involves 3 steps as described below.

### Step A: Preparation of (4-[1-(4-ethoxycarbonylmethoxy-phenyl)-1-methyl-ethyl]-phenoxy)-acetic acid ethyl ester (diester)

Potassium carbonate (41.4 g) was added to solution of BPA in acetone (22.8 g in 310 ml acetone) at room temperature. Sodium iodide (45.0 g) was charged to the above mixture. Ethylbromo acetate (52.0 g) was added drop-wise to the above mixture at room temperature. The resultant mixture was then refluxed at 65°C (bath temperature; reaction temperature at 55°C) for 36 hrs. Acetone was distilled off under vacuum at 50°C under 100 mm Hg. Water (400 ml) was added to the residue and the resultant mixture was extracted with dichloromethane (2x300 ml; 1x100 ml). The organic layer was washed with aqueous sodium hydroxide solution (5 percent solution; 5x200 ml) followed by washing with water (2x300 ml) until the washes indicated a neutral pH. The organic phase was dried over anhydrous sodium sulfate. The dichloromethane was then removed at 50°C under reduced pressure (400 mm Hg). The product obtained was an oily material weighing 34.7 g.

### Step B Preparation (4-[1-(4-carboxymethoxy-phenyl)-1-methyl-ethyl]-phenoxy)-acetic acid (diacid)

A solution of diester (34.7 g; obtained in Step A) in tetrahydrofuran (290 ml) and ethanol (290 ml) was taken in a reaction vessel. An ethanolic solution of KOH (19.7 g dissolved in 290 ml ethanol) was added to the above solution at room temperature. The resultant milky solution was stirred for 19 hours at room temperature and the solvent was removed under reduced pressure (60°C at 10 mm Hg). The solid obtained was dissolved in water (400 ml) and washed with ether (150 ml). The aqueous layer was cooled to 10°C and dilute HCl (4.5 percent, 100 ml) was added to bring the pH of the aqueous layer to 4 to 5. The resulting mixture was stirred in an ice bath (0 to 5°C) for 1 hr. The resulting solid was filtered and washed with chilled water (250 ml) until the washings indicated neutral pH. The crude diacid was recrystallized in methanol and dried to get 18.2 g of product.

### Preparation of acid chloride

A mixture of (4-[1-(4-carboxymethoxy-phenyl)-1-methyl-ethyl]-phenoxy)-acetic acid (diacid; 18.2 g) and dicholoroethane (80 ml) was stirred at room temperature. Thionyl chloride (23 ml) was added drop-wise to the above mixture followed by the addition of dimethylformamide (2 drops) at room temperature. The resultant mixture was refluxed at 90°C for 4 hours. The solvent was removed at reduced pressure below 60°C. Dicholoroethane (30 ml) was added and then distilled off to remove traces of thionyl chloride. The product obtained was an oily substance with a weight of 20.2 g.

### Step C: Preparation of N-(4-hydroxy-phenyl)-2-[4-[1-[4-[(4-hydroxy-phenylcarbamoyl)-methoxy]-phenyl]-1-methyl-ethyl]-phenoxy]-acetamide.

Dimethylformamide (50 ml.), p-amino phenol (13.0 g), and triethylamine (25 ml) were charged to a 250 ml round bottom flask at room temperature. The contents of the flask were cooled to 2 to 5°C and a solution of the acid chloride prepared in Step B (20.2 g acid chloride dissolved in 35 ml dimethylformamide) was added drop wise over a period of 30 minutes. The resultant reaction mixture was stirred at 10°C for 1 hr and at room temperature for 20 hours. The reaction mixture was then cooled to 2 to 5°C and poured on 700 g ice. The resultant mixture was acidified with dilute HCl (10 percent; 10 ml). The solid was then filtered to give a product weighting 85 g (wet weight). The wet product was directly taken for purification.

### Purification of N-(4-hydroxy-phenyl)-2-[4-[1-[4-[(4-hydroxy-phenylcarbamoyl)- , methoxy]-phenyl]-1-methyl-ethyl]-phenoxy]-acetamide.

The crude solid was dissolved in 400 ml of a 10 percent sodium hydroxide solution and stirred at room temperature for 30 minutes. The resultant mixture was acidified with dilute HCl (18 percent, 250 ml) and extracted with ethyl acetate. The solution was filtered through cotton to remove any sticky material. The solvent was distilled off and hexane (100 ml) was added to the residue and the resultant mixture stirred for 20 minutes. The obtained solid was filtered and recrystallized twice with methanol/water (6 parts methanol : 1 part water) to get a product weighing 8 g and having a melting point 206°C.

As can be seen from the foregoing examples a dihydroxy aromatic compound having Formula (I) can be readily prepared as shown in Examples 1, 2 and 3.

The following examples relate to polymers made using the dihydroxy aromatic compounds described herein. The molecular weight (Mw) of the polymer was determined by gel permeation chromatography (GPC) on a Shimadzu system, using chloroform at 35°C through a PLgel 5µm (10³ Angstrom and 10⁵ Angstrom) column and a UV detector at 254 nanometers and compared relative to polystyrene standards. The glass transition temperature (Tg) of the polymer was analyzed on a DSC2920 equipment from TA and values are in °C. The polydispersity index (PDI) is the ratio of the weight average molecular weight to the number average molecular weight. The PDI is measured by dissolving the polymer sample (approximately 10milligrams (mg)) in 1 ml of hexafluoroisopropanol (HFIP) and made up with 9 ml of chloroform (CHCl₃). In Example 10 the mobile phase consists of 5 volume percent HFIP in 95 volume percent CHCl₃. The chemical resistance of the polymer was assessed by conducting the 'Drop Test' method, where a drop of the test solvent (acetone, methyl ethyl ketone, toluene, or ethanol) was placed on a polymer film (cast from a 10 wt. percent solution of polymer in chloroform). After 1 minute, the drop was wiped off and the chemical resistance was assessed through a visual inspection of the surface for any defects (haziness, sticky residue) and labeled as pass or fail.

### EXAMPLES 4-11

### Preparation of N-(4-hydroxy-phenyl)-2-[4-[(4-hydroxy-phenylcarbomoyl)-methoxy]-phenoxy]-acetamide (HQ-BAAP)-bisphenol A (BPA) copolymer.

The general procedure employed for the preparation of the copolymer is given below. A glass reactor tube was passivated with 0.1 N HCl overnight. The tube was then washed with deionized water a few times followed by water purified using a Milli-Q system and then washed with acetone. The tube was then dried with air. Bismethyl salicyl carbonate (bMSC; 20 g; 0.0606 moles), HQ-BAAP (2.4g; 0.0059 moles), and bisphenol A (12.09 g; 0.0529 moles) were added to the glass tube. Sodium hydroxide (3.53 micrgrams, 0.088x10⁻⁶ moles) and tetramethyl ammonium hydroxide (TMAH; 536 micro grams, 5.88x10⁻⁶ moles) were added to the reaction mixture. The mixture was then heated to 180°C. The mixture was stirred constantly at this temperature for 6 minutes. The temperature was then raised to 260°C and the pressure decreased to 101 x 10³ newtons per meter square atmosphere. After 6 minutes, the pressure was gradually decreased to 10 x 10³ newtons per square meter. After 10 minutes at this temperature and pressure, the temperature was further raised to 280°C and the pressure was reduced to 0 Newtons per square meter. The methylsalicylate byproduct was constantly removed throughout the reaction. After 10 minutes at these final conditions, the reactor was brought back to atmospheric pressure (using N₂) and the contents were removed. A clear transparent polymer was obtained. The Tg of the polymer samples was in the range of 140 to 145°C. The melting point of the monomer was determined to be 270°C. Table 1 indicates the molecular weight and polydispersity index (PDI) of different samples prepared as described above having a HQ-BAAP to BPA ratio of 10:90.

**Table 1.**

| Example No | Mw | PDI | Tg °C |
|---|---|---|---|
| 4 | 32172 | 2.44 | 142 |
| 5 | 32369 | 2.44 | ND |
| 6 | 32577 | 2.34 | 141 |
| 7 | 26468 | 2.3 | ND |
| 8 | 27680 | 2.5 | ND |
| 9 | 49268 | 3.9 | ND |
| 10 | 23663 | 2.3 | ND |
| 11 | 17777 | 2 | ND |

| | | | |
|---|---|---|---|
| ND: not determined | | | |

### Example 12-14

### Preparation of N-(4-hydroxy-phenyl)-2-[4-[(4-hydroxy-phenylcarbomoyl)-methoxy]-2-methyl-phenoxy]-acetamide (MeHQ-BAAP)-bisphenol A (BPA) copolymers.

A similar process as followed for Examples 4-11 was employed for Examples 12-14, except that the HQ-BAAP was replaced with MeHQ-BAAP in varying amounts to obtain polymers with varying comonomer ratios with bisphenol A. The results are included in the Table 2 below

**Table 2.**

| Example | MeHQ-BAAP/BPA | Mw | PDI | Tg |
|---|---|---|---|---|
| 12 | 10/90 | 34000 | 2.6 | 132 |
| 13 | 25/75 | 79658 | 5.04 | ND |
| 14 | 50/50 | ND | ND | 136 |

| | | | | |
|---|---|---|---|---|
| ND: Not determined | | | | |

Films of a polymer having 50 mol% MeHQ-bAAP/50 mol% BPA were exposed to acetone, methyl ethyl ketone and toluene as described above and showed no stickiness or increased haziness after an exposure time of 1 minute.

### Example 15

### N-(4-hydroxy-phenyl)-2-[4-[(4-hydroxy-phenylcarbomoyl)-methoxy]-phenoxy]-acetamide (HQ-BAAP)-bisphenol A (BPA) copolymer.

Ethylene dichloride, (500 ml.), deionized water (300 ml), HQ-BAAP (4.3 g), bisphenol A (45.7 g), p-cumylphenol (0.67 g), and triethylamine (0.44 ml) were charged to a 2 liter flask equipped with mechanical agitation, condenser, and a caustic vent scrubber. Phosgene (27.5 g) was added at a rate of 2.0 g/minute with vigorous stirring while a 50 wt. percent solution of sodium hydroxide was added at a rate to maintain a pH of 9 to 10. After phosgene addition was complete, the batch was purged with nitrogen for 10 minutes to ensure complete removal of phosgene. Stirring was stopped, but no phase separation was observed. The reactor contents were transferred to a 2 liter separating funnel, and 1N HCl (500 ml) was charged to the funnel in 50 ml increments and the contents shaken and vented after each acid addition. After the acidification, the phases were allowed to separate on standing.

The resultant organic phase containing polymer was washed with 1N HCl (1x500ml) and then with deionized water (3x500ml). The washed polymer solution was then slowly fed into approximately 2 liter of hot water with rapid stirring to flash off the ethylene dichloride, and the resulting white polymer was isolated by filtration and dried in air at 110°C overnight to provide a 5 mole percent HQ-BAAP polymer.

### Example 16

Methylene dichloride (500 ml.), deionized water (300 ml), HQ-BAAP (32.1 g), bisphenol A (17.92 g), p-cumylphenol (0.50 g), and triethylamine (0.33 ml) were charged to a 2 liter flask equipped with mechanical agitation, condenser, and a caustic vent scrubber. Phosgene (20.4 g) was added at a rate of 1.0 g/minute with vigorous stirring while a 50 wt. percent solution of sodium hydroxide was added at a rate to maintain a pH of 9 to 10. After phosgene addition was complete, the batch was purged with nitrogen for 10 minutes to ensure complete removal of phosgene. Stirring was stopped, but no phase separation was observed. The reactor contents were transferred to a 2 liter separating funnel, and 1N HCl (500 ml) was charged to the funnel in 50 ml increments and the contents shaken and vented after each acid addition. After the acidification, the phases were allowed to separate on standing.

The resulting organic phase containing polymer was washed with 1N HCl (1x500ml) and then with deionized water (3x500ml). The washed polymer solution was then slowly fed into approximately 2 liter of hot water with rapid stirring to flash off the methylene dichloride, and the resulting white polymer was isolated by filtration and dried in air at 110°C overnight to provide a 50 mole percent HQ-BAAP polymer.

### Example 17

Methylene dichloride (200 ml.), deionized water (300 ml), HQ-BAAP (4.87 g), bisphenol A (18.79 g), p-cumylphenol (0.30 g), and triethylamine (0.13 ml) were charged to a 2 liter flask equipped with mechanical agitation, condenser, and a caustic vent scrubber. Phosgene (12.2 g) was added at a rate of 1.0 g/minute with vigorous stirring while a 50 wt. percent solution of sodium hydroxide was added at a rate to maintain a pH of 9 to 10. After phosgene addition was complete, the batch was purged with nitrogen for 10 minutes to ensure complete removal of phosgene. Stirring was stopped, but no phase separation was observed. The reactor contents were transferred to a 2 liter separating funnel, and 1N HCl (500 ml) was charged to the funnel in 50 ml increments and the contents shaken and vented after each acid addition. After the acidification, the phases were allowed to separate on standing.

The resulting organic phase containing polymer was washed with 1N HCl (1x500ml) and then with deionized water (3x500ml). The washed polymer solution was then slowly fed into approximately 2 liters of hot water with rapid stirring to flash off the methylene dichloride, and the resulting white polymer was isolated by filtration and dried in air at 110°C overnight to provide a 50 mole percent HQ-BAAP polymer.

While typical embodiments have been set forth for the purpose of illustration, the foregoing descriptions should not be deemed to be a limitation on the scope herein. Accordingly, various modifications, adaptations, and alternatives may occur to one skilled in the art without departing from the spirit and scope herein.

## Claims

1. A process comprising:
reacting a compound of Formula (III) with a compound of Formula (IV) in the presence of first base to produce a compound of Formula (V) 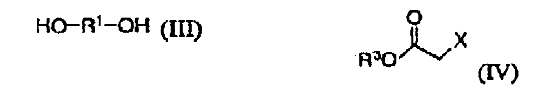 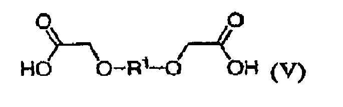
reacting the compound of Formula (V) with a halogenating agent in the presence of a first catalyst to produce a compound of Formula (VI) 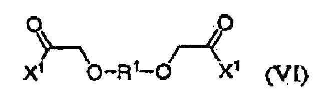
and reacting the compound of Formula (VI) with a compound of Formula (VII) in the presence of a second base to produce a dihydroxy aromatic compound of Formula (I) 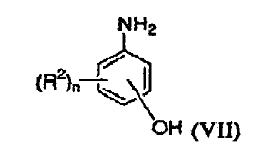 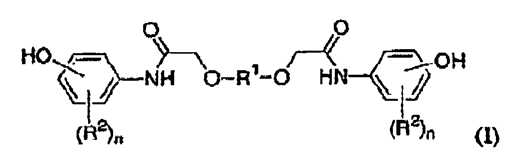 wherein R¹ is an aromatic divalent functionality having 6 to 20 carbons, R² can be the same car different at each occurrence and is independently at each occurrence selected from the group consisting of a cyano functionality, a nitro functionality, a halogen, an aliphatic functionality having 1. to 10 carbons, a cycloaliphatic functionality having 3 to 10 carbons and an aromatic functionality having 6 to 10 carbons, R³ is a hydrogen or an aliphatic functionality having 1 to 10 carbons, X and X¹ arc both independently a halogen selected from the group consisting of chlorine and bromine, and "n" is an integer having a value 0 to 4.

2. The process of Claim 1, further comprising
hydrolyzing a compound of Formula (XIV) in the presence of alkali metal hydroxide or alkali metal carbonate 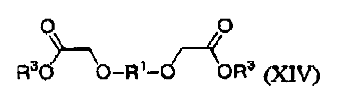 to provide a compound of Formula (V) 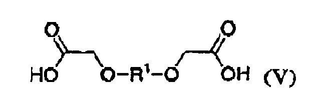 wherein R¹ is an aromatic functionality having 6 to 60 carbons, and R³ is selected from the group consisting of an aliphatic functionality having 1 to 10 carbons.

3. The process of Claim 1 wherein the aromatic divalent functionality R¹ is derived from a dihydroxy aromatic compound having Formule (XIV) 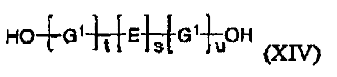 wherein each G¹ is independently at each occurrence an aromatic radical having 6 to 20 carbons; E is independently at each occurrence a cycloaliphatic radical having 3 to 20 carbons, an aromatic radical having 6 to 20 carbons, an aliphatic radical having 1 to 20 carbons, a sulfur-containing linkage, a phosphorus-containing linkage, or an oxygen atom; "t" is a number greater than or equal to one and less than or equal to 10,000, "s" is either zero or one; and "u" is a whole number from zero to 10,000.

4. The process of any of the preceding claims wherein the compound of Formula (111) is hydroquinone, methyl hydroquinone, resorcinol or bisphenol A.

5. The dihydroxy aromatic compound produced by the process of any of the preceding claims,

6. A polymer comprising at least one structural unit derived from the dihydroxy aromatic compound of Claim 5.

7. The polymer of claim 6, wherein the polymer is a polycarbonate, a polyester, a copolyestercarbonate, a polyurethane or an epoxide-containing polymers.

8. The polycarbonate of claim 7 comprising repeating structural carbonate units of the Formula (XIX) 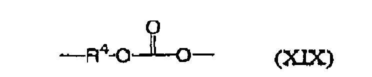 wherein at least 60 percent of the total number of R⁴ groups are aromatic functionalities and the balance thereof are aliphatic, alicyclic, or aromatic functionalities and further wherein at least one R⁴ group is a structural unit derived from a dihydroxy aromatic compound of Formula (1).

9. The polycarbonate of claim 8, further comprising structural units derived from bisphenol A.

10. The polycarbonate, the polyester, or copolyester carbonate of claim 7, further comprising at least one structural unit having Formula (XVI), Formula (XVII) and Formule (XVIII)

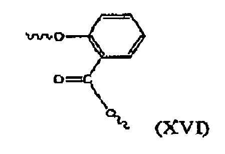

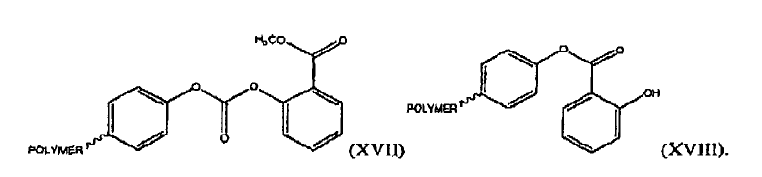

## Patentansprüche

1. Ein Verfahren, das folgendes umfasst:
Reagieren einer Verbindung der Formel (III) mit einer Verbindung der Formel (IV) in der Anwesenheit einer ersten Base, um eine Verbindung der Formel (V) herzustellen 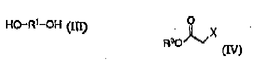 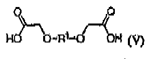
Reagieren der Verbindung der Formel (V) mit einem Halogenierungsmittel in der Anwesenheit eines ersten Katalysators, um eine Verbindung der Formel (VI) zu erzeugen, 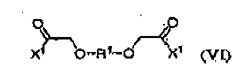
und Reagieren der Verbindung der Formel (V1) mit einer Verbindung der Formel (VII) in der Anwesenheit einer zweiten Base, um eine aromatische Dihydroxy-verbindung der Formel (I) herzustellen 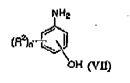 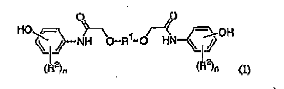 worin R¹ eine aromatische divalente Funktionalität ist mit 6 bis 20 Kohlenstoffen, R² kann bei jedem Vorkommen gleich oder unterschiedlich sein, und ist unabhängig bei jedem Vorkommen gewählt aus der Gruppe bestehend aus einer Cyano-Funktionalität, einer Nitro-Funktionalität, einem Halogen, einer aliphatischen Funktionalität mit 1 bis 10 Kohlenstoffen, einer cycloaliphatischen Funktionalität mit 3 bis 10 Kohlenstoffen, und einer aromatischen Funktionalität mit 6 bis 10 Kohlenstoffen, R¹ ist ein Wasserstoff oder eine aliphatische Funktionalität mit 1 bis 10 Kohlenstoffen, X und X' sind beide unabhängig ein Halogen gewählt aus der Gruppe bestehend aus Chlor und Brom, und "n" ist eine ganze Zahl mit einem Wert von 0 bis 4.

2. Das Verfahren von Anspruch 1, das weiter folgendes umfasst:
Hydrolysieren einer Verbindung der Formel (XIV) in der Anwesenheit von Alkalimetallhydroxid oder Alkalimetallcarbonat 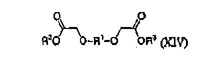
um eine Verbindung der Formel (V) bereitzustellen 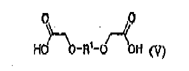
worin R¹ eine aromatische Funktionalität ist mit 6 bis 60 Kohlenstoffen, und R³ ist gewählt aus der Gruppe bestehend aus einer aliphatischen Funktionalität mit 1 bis 10 Kohlenstoffen.

3. Das Verfahren von Anspruch 1, worin die aromatische divalente Funktionalität R¹ abgeleitet ist von einer aromatischen Dihydroxy-Verbindung mit der Formel (XIV) 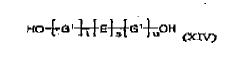 worin jedes G¹ unabhängig bei jedem Vorkommen ein aromatisches Radikal ist mit 6 bis 20 Kohlenstoffen; E ist unabhängig bei jedem Vorkommen ein cycloaliphatisches Radikal mit 3 bis 20 Kohlenstoffen, ein aromatisches Radikal mit 6 bis 20 Kohlenstoffen, ein aliphatisches Radikal mit 1 bis 20 Kohlenstoffen, eine Schwefel-enthaltende Bindung, eine Phosphorenthaltende Bindung oder ein sauerstoffatom; "t" ist eine Zahl größer als oder gleich eins und weniger als oder gleich 10000; "s" ist entweder null oder eins; und "u" ist eine ganze Zahl von null bis 10000.

4. Das Verfahren von irgendeinem der vorhergehenden Ansprüche, worin die Verbindung der Formel (III) Hydrochinon, Methylhydrochinon, Resorcinol oder Bisphenol A ist.

5. Die aromatische Dihydroxy-Verbindung, hergestellt durch das Verfahren von irgendeinem der vorhergehenden Ansprüche.

6. Ein Polymer, das mindestens eine strukturelle Einheit umfasst, die abgeleitet ist von der aromatische Dihydroxy-Verbindung von Anspruch 5.

7. Das Polymer von Anspruch 6, worin das Polymer ein Polycarbonat, ein Polyester, ein Copolyestercarbonat, ein Polyurethan oder ein Epoxid-enthaltendes Polymer ist.

8. Das Polycarbonat von Anspruch 7, das sich wiederholende strukturelle Carbonateinheiten der Formel (XIX) umfasst 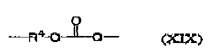 worin mindestens 60 Prozent der Gesamtanzahl von R⁴ Gruppen aromatische Funktionalitäten sind, und der Rest davon sind aliphatische, alicyclische oder aromatische Funktionalitäten, und worin weiter mindestens eine R⁴ Gruppe eine strukturelle Einheit ist, abgeleitet von einer aromatischen Dihydroxy-Verbindung der Formel (I).

9. Das Polycarbonat von Anspruch 8, das weiter strukturelle Einheiten, abgeleitet von Bisphenol A, umfasst.

10. Das Polycarbonat, der Polyester oder das Copolyestercarbonat von Anspruch 7, die weiter mindestens eine strukturelle Einheit der Formel (XVI), Formel (XVII) und Formel (XVIII) umfassen

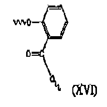

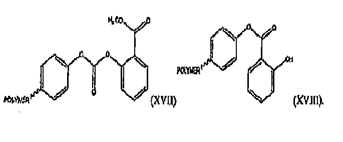

## Revendications

1. Procédé, comprenant:
la mise en réaction d'un composé de Formule (III) avec un composé de Formule (IV), en présence d'une première base, pour produire un composé de Formule (V) : 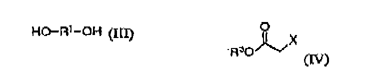 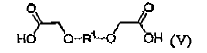
la mise en réaction du composé de Formule (V) avec un agent halogénant, en présence d'un premier catalyseur, pour produire un composé de Formule (VI) : 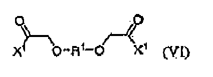
et mise en réaction du composé de Formule (VI) avec un composé de Formule (VII), en présence d'une deuxième base, pour produire un composé dihydroxy aromatique de Formule (I) : 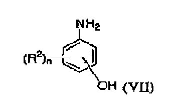 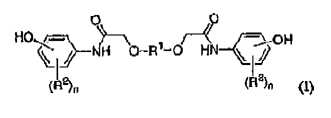 dans lequel R¹ est une fonctionnalité bivalente aromatique ayant de 6 à 20 atomes de carbone, R² peut être identique ou différent, à chaque occurrence, et est indépendamment, à chaque occurrence, sélectionné dans le groupe composé d'une fonctionnalité cyano, une fonctionnalité nitro, un halogène, une fonctionnalité aliphatique ayant de 1 à 10 atomes de carbone, une fonctionnalité cycloaliphatique ayant de 3 à 10 atomes de carbone et une fonctionnalité aromatique ayant de 6 à 10 atomes de carbone, R³ est un hydrogène ou une fonctionnalité aliphatique ayant de 1 à 10 atomes de carbone, X et X¹ sont tous deux, indépendamment, un halogène sélectionné dans le groupe composé de chlorure et bromure, et "n" est un entier ayant une valeur comprise entre 0 et 4.

2. Procédé selon la revendication 1, comprenant en outre :
l'hydrolyse d'un composé de Formule (XIV) en présence d'un hydroxyde de métal alcalin ou un carbonate de métal alcalin 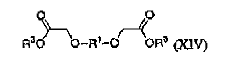
pour fournit un composé de Formule (V) : 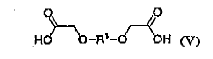 dans lequel R¹ est une fonctionnalité aromatique ayant de 6 à 60 atomes de carbone, et R³ est sélectionné dans le groupe composé d'une fonctionnalité aliphatique, ayant de 1 à 10 atomes de carbone.

3. Procédé selon la revendication 1, dans lequel la fonctionnalité bivalente aromatique R¹ est dérivée d'un composé dihydroxy aromatique présentant la Formule (XIV) : 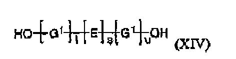 dans laquelle chaque G¹ est, indépendamment, à chaque occurrence, un radical aromatique ayant de 6 à 20 atomes de carbone ; E est, indépendamment, à chaque occurrence, un radical cycloaliphatique ayant de 3 à 20 atomes de carbone, un radical aromatique ayant de 6 à 20 atomes de carbone, un radical aliphatique ayant de 1 à 20 atomes de carbone, une liaison contenant du soufre, une liaison contant du phosphore, ou un atome d'oxygène ; "t" est un nombre supérieur ou égal à un et inférieur ou égal à 10.000, "s" vaut soit zéro soit un ; et "u" est un nombre entier compris dans la fourchette allant de zéro à 10.000.

4. Procédé selon l'une quelconque des revendications précédentes, dans lequel le composé de Formule (III) est hydroquinone, méthyl hydroquinone, résorcinol ou bisphénol A.

5. Composé dihydroxy aromatique produit par le procédé selon l'une quelconque des revendications précédentes.

6. Polymère comprenant au moins une unité structurelle dérivée du composé dihydroxy aromatique selon la revendication 5.

7. Polymère selon la revendication 6, dans lequel le polymère est un polycarbonate, un polyester, un copolyester-carbonate, un polyuréthane, ou un polymère contenant un époxyde.

8. Polycarbonate selon la revendication 7, comprenant des unités structurelles carbonate récurrentes de la Formule (XIX) : 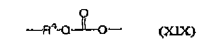 dans laquelle au moins 60 pourcents du nombre total de groupes R⁴ sont des fonctionnalités et le reste sont des fonctionnalité aliphatiques, alicycliques, ou aromatiques, et en outre dans laquelle au moins un groupe R⁴ est une unité structurelle dérivée d'un composé dihydroxy aromatique présentant la Formule (1).

9. Polycarbonate selon la revendication 8, comprenant en outre des unités structurelles dérivées du bisphénol A.

10. Polycarbonate, polyester ou copolyester selon la revendication 7, comprenant en outre au moins une unité structurelle présentant la Formule (XVI), la Formule (XVII) et la Formule (XVIII) :
